(19) Europäisches Patentamt / European Patent Office / Office européen des brevets

(11) **EP 3 313 343 B1**

(12) **EUROPEAN PATENT SPECIFICATION**

(45) Date of publication and mention
of the grant of the patent:
**27.07.2022 Bulletin 2022/30**

(21) Application number: **16747432.9**

(22) Date of filing: **24.06.2016**

(51) International Patent Classification (IPC):
*A61F 13/514* (2006.01)    *B32B 5/02* (2006.01)
*B32B 5/14* (2006.01)    *B32B 5/26* (2006.01)
*B32B 7/02* (2019.01)    *D04H 3/16* (2006.01)

(52) Cooperative Patent Classification (CPC):
**B32B 5/022; A61F 13/514; B32B 5/145;
B32B 5/26; B32B 7/02; D04H 3/007; D04H 3/016;
D04H 3/14; D04H 3/16;** B32B 2307/7242;
B32B 2307/7246; B32B 2307/7265; B32B 2555/02

(86) International application number:
**PCT/CZ2016/000069**

(87) International publication number:
**WO 2016/206659 (29.12.2016 Gazette 2016/52)**

(54) **NONWOVEN WEB WITH ENHANCED BARRIER PROPERTIES**

VLIESSTOFF MIT VERBESSERTEN BARRIEREEIGENSCHAFTEN

BANDE DE NON-TISSÉ À PROPRIÉTÉS BARRIÈRES AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2015 CZ 20150441**

(43) Date of publication of application:
**02.05.2018 Bulletin 2018/18**

(73) Proprietor: **PFNonwovens Czech s.r.o.
669 02 Znojmo (CZ)**

(72) Inventors:
• **MECL, Zdenek
 67181 Novy Saldorf - Sedlesovice (CZ)**
• **KOHUT, Jaroslav
 66902 Znojmo (CZ)**
• **KASPARKOVA, Pavlina
 66902 Znojmo (CZ)**

(74) Representative: **Zemanová, Veronika
Kania, Sedlak, Smola
Patent Attorneys
Mendlovo namesti 1 a
603 00 Brno (CZ)**

(56) References cited:
**WO-A2-2005/005704    WO-A2-2011/131156
US-A1- 2005 215 155**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the invention**

The invention relates to a bulky spunmelt-type nonwoven textile containing several mutually supportive layers with various fibre diameters. The nonwoven textile of this type is intended predominantly for use in the hygiene industry but can be used wherever barrier properties are required - for example, as a material for protective garments in medicine, industry or for use in healthcare.

**Background art**

A nonwoven textile may be utilised in a wide range of applications. Various nonwoven textiles may contain spunbond, meltblown, spunbond ("SMS") layers, where the contained outer textile layers are formed by a thermoplastic spunbond (e.g. from polyolefins) and the inner layers by a thermoplastic meltblown material. The SMS nonwoven textile of this type may contain stable spunbond layers and an inner meltblown layer, which despite being porous has the ability to prevent, for example, rapid seepage of fluids such as for example bodily fluids or solid compounds such as for example superabsorbent powder.

As known in the art, the combination of a stronger spunbond fibre layer and a finer meltblown or nano-fibre layer in SMS-type composites (or multi-layer-type combinations, i.e. SSMMS, SMMMS, SNS) enable the utilisation of the advantages and suppression of the disadvantages of both layer types. Whereas continuous spunbond-type fibres provide mechanical properties (strength, extensibility), fine fibres (for example meltblown) provide the missing barrier properties whilst being protected within the composite against easy mechanical damage. By combining layers of fine and strong fibres within a composite it can be assumed, that the determinant layer for barrier properties is the layer with the finest fibres (and thus the smallest pores).

It is known in the art that layers formed with finer fibres have, at the same fibre packing density, better barrier properties than layers formed with coarser fibres. In the case of spunmelt-type nonwoven textile applications in the hygiene industry, namely for absorptive products, the barrier properties of meltblown-type layers are utilised. The utilisation of layers with a significant share of sub-micron fibres is also known (so-called nano-fibres). There exist many described methods for the production of a layer of fibres with a significant content of sub-micron fibres - for example by utilising special meltblown spinnerets (described for example in patent EP2019875 belonging to Hills), electrospinning (based on the work of professor Jirsak - e.g. US patent 7,585,437 and many others), foil splitting (e.g. a document from Procter and Gamble EP1639173) or for example by utilising rotating equipment (e.g. produced by Fiber Rio or described in for example document US2009232920 belonging to the University of Texas).

Without wanting to be bound by theory, it can be assumed that the fineness of fibres at a constant fibre packing density also significantly correlates with the size of pores in the layer. It can thus be assumed that at a constant fibre packing density it applies that: the finer the fibres, the smaller the cross-section of the pores passing through the fibrous layer and the smaller the level of curvature of the individual passage pores in the vertical direction will be. Thus by this reasoning: the smaller and the more curved the pores, the more complicated will be the seepage of, for example, fluids and this will increase the barrier capacity of the layer against the seepage. This increased barrier may manifest itself, for example, in the form of a higher water column parameter value (a method for measuring the seepage of a fluid through a substrate) or, for example, an increased retention of fluids with a surface tension closer to the surface tension of the material of the fibres. For example in the case of absorptive hygiene products, namely diapers, one of the fundamental functions of the product is its high ability to retain urine within the product, which apart from the sorptive core is provided also by the barrier layers made from a nonwoven textile. For example, an SMS-type nonwoven textile produced from polypropylene has a surface tension of approximately 30 mN/m and inside the diaper it comes into contact with urine which has a surface tension of approx. 40-50 mN/m for adult individuals and in the range of 32-35 mN/m for newborns. Due to the small difference in energy, it can be assumed, that undesirable seepage through the barrier may more easily occur.

Absorptive products such as for example diapers, training pants, incontinence aids and female hygiene products may contain nonwoven textiles performing various functions - one of the significant requirements is specifically to provide a barrier function. It is also known in the art that layers of fine fibres are utilised for increasing barrier properties of such absorptive hygiene products (for example patent application of Procter & Gamble WO2011100407). To achieve a significant increase in barrier properties, it is significant that a narrow distribution of fibres is maintained and to ensure as great a share of sub-micron fibres as possible.

US2005215155 discloses a layered nonwoven textile comprising two identical meltblown layers arranged between spunbond layers with fibers having differing crosssectional shapes.

There is significant demand in the market for the reduction of the basis weight of the used nonwoven textiles whilst retaining their barrier properties, which leads to efforts to increase the barrier property especially of thin light-weight layers.

## Summary of the invention

The aforementioned task according is resolved to the invention by a nonwoven textile with barrier properties comprising of

  a. a first barrier layer A with a theoretical cover coefficient TCC A, and consisting of fibres having a median fibre diameter in the layer of dAm; and
  b. a second barrier layer B with a theoretical cover coefficient TCC B, and consisting of fibres having a median fibre diameter in the layer of dBm; whilst

   i. the first barrier layer A and the second barrier layer B are in direct contact with each other;
   ii. the median diameters of fibres in the first and second layer have a theoretical fibre diameter coefficient x = (dBm-dAm)/dAm, where the theoretical fibre diameter coefficient x is less than or equal to 1 and where the theoretical fibre diameter coefficient x is greater than or equal to 0.25; and
   iii. the sum of the theoretical cover coefficient TCC A and TCC B is greater than or equal to 50%.

Advantageous embodiments of the invention are described in dependent patent claims.

**Definition:** The term **"batt"** herein refers to materials in the form of fibres that are found in the state prior to mutual bonding. The "batt" consists of individual fibres between which a mutual bond is usually not yet formed even though they may be pre-bonded in certain ways, where this pre-bonding may occur during or shortly after the deposition of fibres in the spunmelt process. This pre-bonding, however, still permits a substantial number of the fibres to be freely mobile such that they can be repositioned. The aforementioned "batt" may comprise several layers created by the deposition of fibres from several spinning beams in the spunmelt process, whilst it applies that the distribution of fibre diameters and porosity in the "sublayers" deposited by the individual spinning beams does not exhibit significant variance. Adjacent fibre layers must not necessarily be separated from each other by a sharp transition; individual layers in the bordering region may partially blend together.

The term **"barrier layer"** or **"barrier nonwoven textile"** herein refers to a layer of fibres or a nonwoven textile, which is able to stop the flow of material. For example, to stop a flow of liquid and to retain it in a designated area. For example, the parameter "height of the water column" expresses the ability to stop and retain a polar liquid. For example, the parameter "alcohol repellency" expresses the ability to stop and retain a non-polar liquid.

The terms **"fibre"** and **"filament"** are in this case mutually interchangeable.

For the expression of **"fibre diameter"** SI length units are used - micrometres ($\mu$m) or nanometres (nm). For the purposes of this document, the terms "fibre diameter" and "fibre thickness" are interchangeable. In the event that fibres do not have a circular fibre cross-section, a fibre diameter is assumed that corresponds to the equivalent fibre with a circular fibre cross-section. The terms "number of grams of fibre per 9000 m" (also titre denier or Tden or den) or "number of grams of fibre per 10000 m" (dTex) are used to express the degree of fineness or coarseness of a fibre.

For the purposes of this document, the terms **"median fibre diameter"** or **"median fibre thickness"** are interchangeable with the term **"median fibre diameter in the layer"**, abbreviated to **"median".** Thus, it applies that at least 50% of the fibres have a diameter less than or equal to the value of the median and at least 50% of the fibres have a diameter greater than or equal to the median.

The term "sub-micron fibres" refers to normal fibres with a diameter below 1 micron. Usually, however not necessarily, these fibres are substantially thicker than "nano-fibres" (the diameter of which should be less than 100 nanometres). Sub-micron fibres have a diameter typically greater than approximately 200 nm, frequently greater than approximately 500 nm.

For the purposes of this document, the terms "micron" and "micrometre" are interchangeable and express 1/1,000,000 m (measuring unit is $\mu$m).

The term **"mono-component fibre"** refers to a fibre formed of a single polymer component or single blended polymer component, as distinguished from bi-component or multi-component fibre.

Herein, the term **"compound"** or **"polymer compound"** typically refers to polymer materials contained in the fibre composition. For example when multiple compounds are mixed together in a single composition. This does not exclude the addition of other compounds, typically in smaller amounts (e.g. dyes, process additives, surface modification additives, etc.).

The terms **"two-component fibre"** and **"bi-component fibre"** refer to a fibre having a cross-section comprising two discrete polymer components, two discrete blended polymer components, or one discrete polymer component and one discrete blended polymer component. The term "bi-component fibre" is encompassed within the term "multi-component fibre". A bi-component fibre may have its overall cross-section divided into two or more components consisting of differing components of any shape or arrangement, including, for example, a coaxial arrangement, core-and-sheath arrangement, side-by-side arrangement, "segmented pie" arrangement, etc. The term "main component" refers to the component having the greater share of mass in the fibre. For example, the term "C/S 70/30" refers to a bi-component fibre in the arrangement core-and-sheath, where the core corresponds to 70 % of the mass of the fibre and the sheath to 30 % of the mass of the fibre.

The term **"multi-component"** refers to a fibre having a cross-section comprising of more than one discrete polymer component, more than one blended polymer component, or at least one discrete polymer component and at least one independent blended polymer component. The term "multi-component fibre" thus includes, but is not limited to "bi-component fibre". A multi-component fibre may have its overall cross-section divided into parts consisting of differing components of any shape or arrangement, including, for example, a coaxial arrangement, core-and-sheath arrangement, side-by-side arrangement, radial arrangement, islands-in-the-sea arrangement, etc.

The term **"nonwoven textile"** means a structure in the form of a fleece or fibrous layer formed from directed or randomly oriented fibres, from which initially a batt is formed and which is subsequently mutually interconnected (consolidated). Fibres are mutually bonded by friction, effects of cohesive forces, gluing with bonds or other adhesives or thermoplastically creating a single or multiple cohesive - bonding patterns consisting of bonding imprints formed by a bonding compression and/or the effect of pressure, heat, ultrasound or heat energy or a combination of these effects if necessary. The term does not refer to fabrics formed by weaving or knitting or fabrics using yarn or fibres to form bonding stitches. The fibres may be of natural or synthetic origin and may be staple fibres, continuous fibres or fibres produced directly at the processing location. Standard available fibres have a diameter ranging from approx. 0.0001 mm up to approx. 0.25 mm and are supplied in several different forms: short fibres (also known as staple fibres). Continuous single fibres (so-called filaments or mono-filaments) or bundles of continuous fibres (so-called multi-filaments or cables) and bundles of continuous fibres with a common twist (yarn). A nonwoven textile can be produced using many methods, including technologies such as meltblown, spunbond, spunmelt, spinning from solvents, electrostatic spinning (electrospinning), carding, film fibrillation, melt-film fibrillation, airlaying, dry-laying, wetlaying with staple fibres and various combinations of these and other processes as known in the art. The basis weight of nonwoven textiles is usually expressed in grams per square metre (gsm).

**"Fibre packing density"** $\mu$ is the ratio of the volume of fibres in a given space V to the total volume of the given space Vc at a given pressure. The value $\mu$ is in the range from 0 (empty space) to 1 (completely filled space, does not contain any pores). A person skilled in the art understands that for spunmelt-type nonwoven textiles (spunlaid), compared to other nonwoven textile production technologies, the thickness of the batt is spread across a much smaller range. To simplify, it is possible to correlate a given polymer and its fibre packing density with the basis weight of the given layer - a measuring unit that is widely used for this type of a nonwoven textile characteristic. It can thus, likewise, be assumed that for a given polymer also at a constant basis weight it applies that: the finer the fibres, the smaller will be the diameter of the pores passing through the batt and that the level of curvature of the individual passage pores in the vertical direction will also increase and furthermore that based on the aforementioned principle the barrier capacity of the layer will be increased.

The term **"theoretical fibre packing density coefficient"** or **"TCC"** (theoretical cover coefficient) represents the coverage of a specific measuring unit by fibres and is dependent on the basis weight, density of the material forming the fibre and the cross-section of fibres contained in the nonwoven textile. TCC can thereby be visualised by imagining that from the entire mass contained in the assumed nonwoven textile, a single fibre is created with a circular cross-section corresponding to the median fibre diameter in the layer and that this fibre is then laid out on a surface in such a way that the fibres do not cross over or otherwise overlap. The proportion of the covered area then forms the TCC. The finer the fibres, with all other values remaining constant, the greater is the TCC, and similarly, TCC declines with an increasing density of the material that forms the fibre (with all other values remaining constant).

The TCC is calculated according to the following formula:

$$\text{"theoretical cover coefficient" (TCC) } \% = d * L * 100\%;$$

$$L = 4V / \pi d^2;$$

$$V = m / \rho$$

thus:

$$TCC\ \% = (4 * m * 100\%) / (\pi * \rho * d)$$

wherein: d... median fibre diameter of a given layer = mean of a theoretical fibre (m)

L ... length of fibre in 1 m$^2$ of textile (m/m$^2$)
V ... volume of fibre in 1 m$^2$ of textile (m$^3$/m$^2$)
m ... mass of fibre in 1 m$^2$ of textile (g/m$^2$, corresponds to basis weight)
$\rho$ ... fibre density (mass g/ volume m$^3$; corresponds to the density of the material from which the fibre is produced)

For the purposes of this document, the term **"theoretical fibre diameter coefficient"** x expresses the ratio between the median fibre diameters in adjacent layers. For adjacent layers A, B it is expressed by the relationship:

theoretical fibre diameter coefficient x = (dBm - dAm)/dAm
where: dBm ... median fibre diameter in layer B (nm)
dAm ... median fibre diameter in layer A (nm)

The term **"absorptive hygiene product"** herein refers to products or aids, that absorb or retain bodily excretions, more specifically to products or aids, that are placed against the body or placed in the vicinity of the body of the user for the purpose of absorbing and retaining various bodily excretions. Absorptive hygiene products may include disposable diapers, diaper pants, underwear and pads intended for adults suffering from incontinence, female hygiene products, nursing pads, disposable changing pads, bibs, bandages and similar products. The term "excretions" refers to, in the sense used herein, namely to urine, blood, vaginal secretions, breast milk, sweat and faeces.

## Brief description of the drawings

Exemlary embodiments of the invention are further described in greater detail with reference to drawings, where in Fig. 1 the pair of layers A and B according to the invention is schematically shown; in Fig. 2 through Fig. 3b this pair is shown in combination with other layers; and in Fig. 4a through Fig. 4h there are various types of nonwoven textiles described in examples 1 to 13. Figures 5-7 show construction examples of disposable hygiene products - diapers.

## Description of exemplary embodiments

The subject of the invention is a nonwoven web with enhanced barrier properties even when a relatively small amount of material is used. With surprise it was discovered that an appropriate combination of selected fibres positively affects the overall barrier properties of a nonwoven textile - if it contains two barrier layers A and B, which are characterised by given characteristics, namely the median fibre diameter in layer dAm and dBm and a theoretical cover coefficient TCC for each layer TCC A and TCC B. And, furthermore, when relatively to each other, both layers fulfil the first criteria - theoretical fibre diameter coefficient x is found in the given range, and when in the same way they fulfil the second criteria - that the combined TCC AB (sum of TCC A and TCC B) is found in the given range.
The above mentioned should be viewed in contrast to the conventional approach, where the largest content of the finest fibres is required for an improvement of barrier properties. Without the need to be bound by theory, we believe that a barrier layer with somewhat thicker fibres provides the second barrier layer made from somewhat finer fibres better support and thereby enables better utilisation of the barrier potential of the finer fibres in comparison to a layer containing fibres of the same thickness.
Based on the invention, the nonwoven textile is composed of several fibrous layers differing from each other namely by the median of diameters of the fibres contained within them. For the invention it is significant that this median in the individual layers differs sufficiently but not excessively. With surprise it was discovered, that with an appropriate combination of layers with appropriate differences in the median of the fibres contained in them, synergy occurs, and that contrary to assumption, the barrier effect actually increases. Without the need to be bound by theory, we assume that in the case of the combination of layers with excessively small difference, the layers amalgamate into a single practically homogeneous layer. Layers with an excessively high difference most probably do not fully utilise the potential of the support layer - the pore size in this layer is, when compared to the fibre size in the barrier layer, too large and may result in the mechanical damage of the barrier layer sooner than the potential barrier properties of this given layer are utilised.
For the purpose of eliminating fibre layers, the fibre diameters of which excessively differ from each other, a "theoretical fibre diameter coefficient x" was introduced. We believe that when this value is too great, the finer fibres will not find sufficient support in the fibres of the second barrier layer. Similarly, when the difference is too small, such as, for example, when both layers have fibres of the same diameter, the layers will blend into a single, practically homogeneous layer.

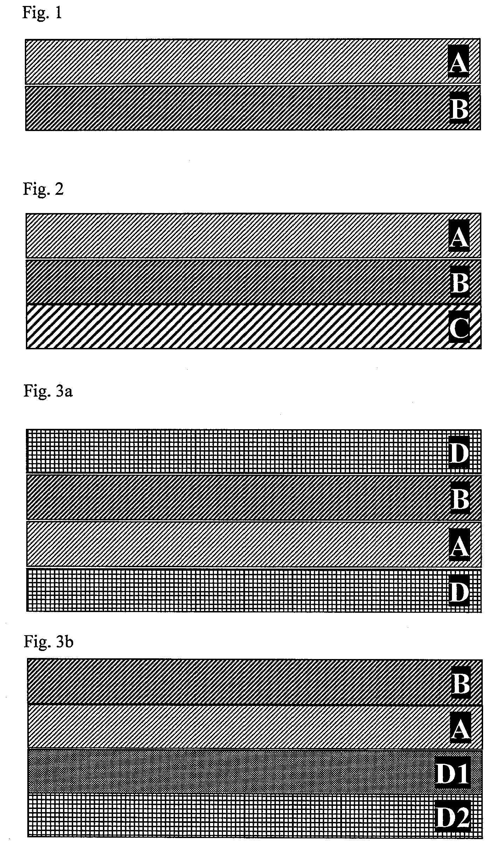

The nonwoven textile according to the invention contains a barrier layer A and another adjacent layer B (fig. 1). Without the need to be bound by theory, we assume that the layer A forms the basic barrier. Layer A comprises fibres with a smaller median diameter than the layer B. It is assumed that the layer B, apart from others, also has another supportive function in the nonwoven textile, wherein it supports the fibres in layer A and enables better utilisation of the barrier potential. An appropriate ratio of medians of fibre diameters in layers A and B expresses the theoretical fibre diameter coefficient x as expressed by the relationship:

$$x = (\text{median fibre diameter in layer B dBm [nm]} - \text{median fibre diameter in layer A}$$

$$\text{dAm [nm]}) / (\text{median fibre diameter in layer A dAm [nm]}).$$

For the invention it is significant that the value of coefficient x is at least 0.25, preferably 0.3 and concurrently less than or equal to 1.0.

If the amount of fibres forming the layer is too small, then the fibres are deposited far from one another and the porosity of the layer is greater than would correspond to the diameter of the fibres. Without the need to be bound by theory, we assume that the critical threshold for achieving the required barrier properties is, in the case of a single layer, a coverage of at least 70%, preferably a coverage of 130% according to the "theoretical cover coefficient (TCC)" and at least 20% coverage for each layer, better yet 25% coverage for each layer, preferably at least 30% coverage for each layer in the case of the described combination of layers A and B, where mutual synergies may occur - layers may partially blend together and the thinner fibres of layer A may reduce the pores created by the coarser fibres of layer B. It still, nevertheless, applies that the sum of the TCC values for layers A, B is at least 50%, better yet at least 60%, better yet at least 70%, preferably at least 100%.

For example, for polypropylene fibres (polymer density of 0.93 g/cm$^3$) in the sub-micron and micron range (0.5-2 microns), the lower limit for the fibre basis weight of layer A can be considered (calculation performed for a fibre diameter of 1 micron) to be at least approx. 0.15 g/m$^2$, better yet 0.18 g/m$^2$, preferably 0.22 g/m$^2$ and for the layer of the stronger fibres B (calculation performed for coefficient x = 0.3, that is a median fibre diameter in layer B is 1.3 microns) at least 0.19 g/m$^2$, better yet 0.24 g/m$^2$, preferably 0.28 g/m$^2$. Whilst for the combination of layers it must always apply that the sum of TCC A and TCC B values for layers A, B is at least 50%. If for example, layer A with a basis weight of 0.2 g/m$^2$ consists of the aforementioned polypropylene fibres with a density of 0.93 g/cm$^3$ and the median fibre diameter in the layer is 1 micron (corresponding to a cover coefficient of 27.4%), and if coefficient x = 0.3 is considered, the median fibre thickness in layer B is 1.3 microns and the condition that the overall sum of TCC is at least 50% is met, then the cover coefficient of layer B must be at least 22.6%, which for polypropylene of the aforementioned density equates to a basis weight of 0.21 g/m$^2$. Therefore, the total basis weight of layers AB is 0.41 g/m$^2$.

For example, in the case of spunbond fibres with a thickness in the one to two denier range (produced from polypropylene with a density of 0.93 g/cm$^3$), the lower limit for the fibre basis weight of layer A can be considered (calculation performed for a fibre diameter of 14.5 microns - corresponds to approx 1.4 den) to be at least approx. 2.4 g/m$^2$, better yet 3.7 g/m$^2$, preferably 4.3 g/m$^2$ and for the layer of the stronger fibres B (calculation performed for coefficient x = 0.5, that is a median fibre diameter in layer B is 21.8 microns) at least 3.6 g/m$^2$, better yet 4.4 g/m$^2$, preferably 5.2 g/m$^2$. The TCC sum condition must apply as in the preceding example.

When calculating coverage, it is necessary to consider that one layer, in the sense of the described invention, may be produced using a single or multiple spinning beams.

Further examples of TCC values for fibres of specific material compositions are provided in the following tables. A person skilled in the art will easily understand that due to the effect of various polymer densities, the provided tables cannot be applied universally but rather must be recalculated for the specific material type. TCC meeting the advantageous configuration (20-800%) is in bold type.

| Fibre diameter | | Basis weight of a mono PP fibre layer (g/m$^2$) Fibre density = 0.93 g/cm$^3$ | | | | | | | | | |
|---|---|---|---|---|---|---|---|---|---|---|---|
| den | microns | 0.1 | 0.2 | 0.3 | 0.5 | 1 | 1.5 | 2 | 3 | 10 | 30 |
| - | 0.5 | **27%** | **55%** | **82%** | **137%** | **274%** | **411%** | **548%** | 821% | 2738% | 8214% |
| - | 1 | **14%** | **27%** | **41%** | **68%** | **137%** | **205%** | **274%** | **411%** | 1369% | 4107% |
| - | 1.5 | 9% | 18% | **27%** | **46%** | **91%** | **137%** | **183%** | **274%** | 913% | 2738% |
| - | 2 | 7% | 14% | **21%** | **34%** | **68%** | **103%** | **137%** | **205%** | **685%** | 2054% |

(continued)

| Fibre diameter | | Basis weight of a mono PP fibre layer (g/m²) Fibre density = 0.93 g/cm³ | | | | | | | | | |
|---|---|---|---|---|---|---|---|---|---|---|---|
| den | microns | 0.1 | 0.2 | 0.3 | 0.5 | 1 | 1.5 | 2 | 3 | 10 | 30 |
| 0.8 | 11 | 1% | 2% | 4% | 6% | 12% | 19% | 25% | 37% | 124% | 373% |
| 1.08 | 13 | 1% | 2% | 3% | 5% | 11% | 16% | 21% | 32% | 105% | 316% |
| 1.2 | 13.5 | 1% | 2% | 3% | 5% | 10% | 15% | 20% | 30% | 101% | 304% |
| 1.4 | 14.5 | 1% | 2% | 3% | 5% | 9% | 14% | 19% | 28% | 94% | 283% |
| 1.64 | 15.8 | 1% | 2% | 3% | 4% | 9% | 13% | 17% | 26% | 87% | 260% |
| 2.04 | 17 | 1% | 2% | 2% | 4% | 8% | 12% | 16% | 24% | 81% | 242% |
| 2.54 | 20 | 1% | 1% | 2% | 3% | 7% | 10% | 14% | 21% | 68% | 205% |

| Fibre diameter | | Basis weight of a bico PLA/PP (C/S 70/30) fibre layer (g/m²) Fibre density = 1.168 g/cm³ (70% *1.27 + 30%*0.93) | | | | | | | | | |
|---|---|---|---|---|---|---|---|---|---|---|---|
| den | microns | 0.1 | 0.2 | 0.3 | 0.5 | 1 | 1.5 | 2 | 3 | 10 | 30 |
| - | 0.5 | 22% | 44% | 65% | 109% | 218% | 327% | 436% | 872% | 1308% | 4360% |
| - | 1 | 11% | 22% | 33% | 55% | 109% | 164% | 218% | 436% | 654% | 2180% |
| - | 1.5 | 7% | 15% | 22% | 36% | 73% | 109% | 145% | 291% | 436% | 1453% |
| - | 2 | 5% | 11% | 16% | 27% | 55% | 82% | 109% | 218% | 327% | 1090% |
| 0.8 | 11 | 4% | 7% | 11% | 18% | 36% | 55% | 73% | 145% | 218% | 727% |
| 1.08 | 13 | 1% | 2% | 3% | 4% | 8% | 13% | 17% | 34% | 50% | 168% |
| 1.2 | 13.5 | 1% | 2% | 2% | 4% | 8% | 12% | 16% | 32% | 48% | 161% |
| 1.4 | 14.5 | 1% | 2% | 2% | 4% | 8% | 11% | 15% | 30% | 45% | 150% |
| 1.64 | 15.8 | 1% | 1% | 2% | 3% | 7% | 10% | 14% | 28% | 41% | 138% |
| 2.04 | 17 | 1% | 1% | 2% | 3% | 6% | 10% | 13% | 26% | 38% | 128% |
| 2.54 | 20 | 1% | 1% | 2% | 3% | 5% | 8% | 11% | 22% | 33% | 109% |

An upper limit for the basis weight is not, from the viewpoint of the functionality of the invention, limited and a person skilled in the art will easily understand that with a growing % of coverage according to TCC, the barrier of the individual layers increases and a relative reduction of the described increase in barrier properties may occur as a result of the combination of layers according to the invention.

Without the need to be bound by theory, it is assumed that the upper limit in the area particularly suitable for the implementation of the invention is perceptible up to a TCC of no more than 800 % for every layer, better yet no more than 600 % for every layer , better yet no more than 400 % for every layer and preferably no more than 200 % for every layer in the case of the described A and B layer combination, where mutual synergies may occur.

For example, for polypropylene fibres (polymer density of 0.93 g/cm³) in the sub-micron and micron range (0.5-2 microns), the upper limit for the especially advantageous configuration of the invention (calculation performed for a fibre diameter of 2 microns) can be considered to be a basis weight of fibres in layer A of no more than 12 g/m², better yet 9 g/m², better yet 6 g/m², preferably 3 g/m² and for the layer of the stronger fibres B (calculation performed for coefficient x = 1, that is a median fibre diameter in layer B is 4 microns) of no more than 23 g/m², better yet 18 g/m², better yet 12 g/m², preferably 6 g/m².

For example, in the case of spunbond fibres with a diameter in the one denier range (produced from polypropylene with a density of 0.93 g/cm³), the upper limit for the advantageous configuration of the invention can be considered (calculation performed for a fibre diameter of 13 microns - corresponds to approx. 1 den) to be no more than approx. 76 g/m², better yet 57 g/m², better yet 38 g/m², preferably 19 g/m² and for the layer of the stronger fibres B (calculation performed for coefficient x = 0.5, that is a median fibre diameter in layer B is 19.5 microns) be no more than 114 g/m², better yet 85 g/m², better yet 57 g/m², preferably 28 g/m².

Layers A and B may, for example, be composed of meltblown fibres. In such a case, layers A, or both layers A and B may contain a certain amount of fibres with a diameter under 1 micron. For example, layer A may contain up to 100% of fibres with a diameter under 1 micron, or may contain up to 50% of fibres with a diameter under 1 micron, or may contain up to 25% of fibres with a diameter under 1 micron, or may contain up to 20% of fibres with a diameter under 1 micron, or may contain up to 10% of fibres with a diameter under 1 micron, or may be composed of fibres only with a diameter over 1 micron. A person skilled in the art understands that if there are fibres present in layers A and B with a diameter under 1 micron, layer A will generally contain more of them than layer B.

In a non-claimed embodiment, layers A and B may, for example, also be composed of exclusively spunbond fibres.

The nonwoven textile according to the invention may contain a further layer C adjoining layer B and relating to layer B in the same manner as layer B to layer A (fig. 2).

The nonwoven textile according to the invention may contain a number of other layers mutually adjoining each other having the same relationship to one another as layers A and B.

The nonwoven textile according to the invention may also contain several combinations of layers having the same mutual relationship as layers A and B, whilst the individual pairs may and may not adjoin one another.

The nonwoven textile according to the invention contains one or multiple other layers D, which may contain any type of fibres and adjoin to the individual layers A or B, whilst the fibres in layer D do not have a described relationship with the fibres in layers A, B. For the nonwoven textile according to the invention it is important that it contains at least one combination of layers A and B (fig. 3).

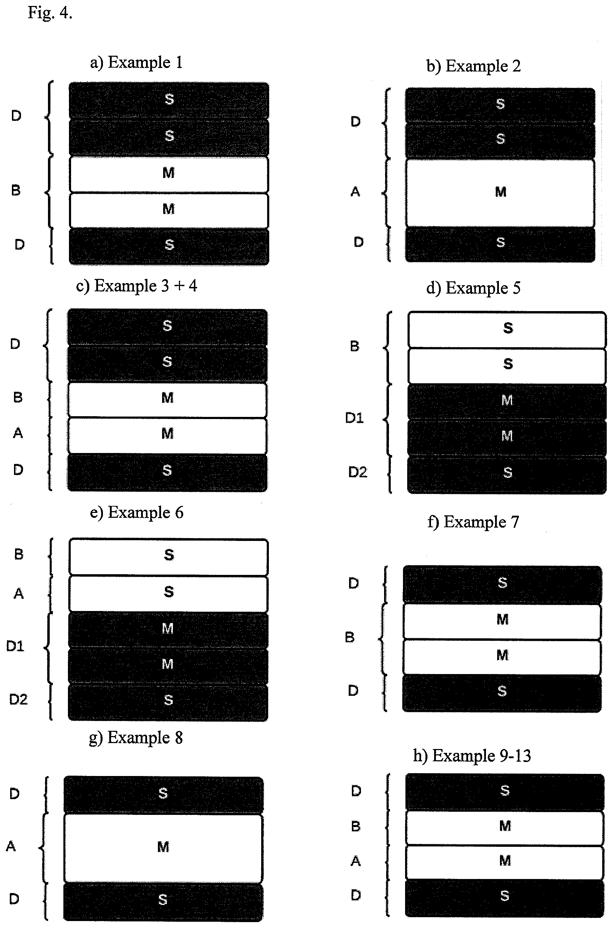

For example, composition DABD (fig. 3a) may, for example, be created in such a way that layers A and B are composed of meltblown fibres and both D layers are composed of spunbond fibres (for example, composition SSMMS shown in figure 4-c). In this case it generally applies that layers D provide primarily mechanical properties and layers AB provide primarily barrier properties. A person skilled in the art understands that this is an advantageous configuration (given by layers AB) of a so-called SMS composite.

For example, composition BAB may, for example, be formed purely from spunbond fibres. The described composition brings both advantages of improved barrier properties as well as a symmetrical material.

For example, composition BAD1D2 (fig. 3b) may, for example, be created by layers A, B and D2 composed of spunbond fibres and layer D1 being composed of meltblown fibres (fig. 4-e). Also in this case, it still applies that spunbond fibres provide primarily mechanical properties. However, the advantageousness of the combination of layers A and B according to the invention provides an improvement in barrier properties, despite the main component of barrier properties being borne by layer D1 composed of meltblown fibres. A person skilled in the art will appreciate that the invention may, independently of itself, be used in both spunbond as well as meltblown fibre layers to achieve an improvement in the barrier properties of both - by creating, for example, composition B1A1B2A2D. A person skilled in the art can easily imagine many other suitable combinations of layers A, B and D.

The nonwoven textile according to the invention contains at least one pair of mutually adjoining fibrous layers differing from one another namely by the length of the fibres used in them as shown in fig. 1. The entire structure is reinforced in such a way that the nonwoven textile achieves the required mechanical properties, whilst the barrier function of the material is thereby not significantly impaired.

The nonwoven textile according to the invention has increased barrier properties, i.e. it can better block the flow of material for a longer time. For example, in the case of a barrier against seepage of fluids, a nonwoven textile in a hydrophobic configuration is able to better stop and retain polar fluids, solutions and their blends. For example, when used as a part of a diaper, it is able to stop the flow of urine and to retain urine in the area of the sorptive core to enable the sorptive core to draw it into itself. For example, in the case of a barrier against the seepage of fluids, a nonwoven textile in an alcohol-repulsive configuration is able to better stop and retain polar and non-polar fluids, solutions and their blends. For example, when used as a part of a protective garment, it is able to prevent a liquid solvent from seeping through to the skin - for example, when used as a protective garment in healthcare, it must not permit seepage of, for example, blood, bodily excretions or agents, used for example, for disinfection.

The barrier properties of the nonwoven textile according to the invention do not mean that the textile forms an absolute barrier. For example, in products worn on the body (for example, hygiene absorptive products or protective garments) it may, in certain applications, be suitable to form a barrier against liquids, while maintaining a certain ability for gases to pass through, meaning that the products are air permeable and thus comfortable to wear. A person skilled in the art will understand that the described barrier properties differ from the properties required in filtration applications, where a current of air or fluid is passing through the filter and the filter captures only undesirable impurities (for example, dust in the air current).

The nonwoven textile according to the invention has increased barrier properties.

The nonwoven textile may be produced predominantly from polymer fibres. As examples of materials suitable for the production of nonwoven textiles namely fibres from polymer materials that may be mentioned are polyolefins, polyesters or polyamides. More specifically then, for example, polypropylene (PP), polyethylene (PE), polyethylene terephthalate (PET), polybutylene terephthalate (PBT), polytrimethylene terephthalate (PTT) and/or co-polymers of these materials. Fibres for the production of nonwoven textiles may, for example, be composed of so-called biopolymers such as aliphatic polyesters, thermoplastic polysaccharides or other polymers of biological origin or renewable polymers.

The above mentioned compounds may be present independently, in the form of various co-polymers or blends, possibly the composition may contain other substances such as adjuncts (for example, dyes, functional additives, etc.) or modifiers. The purpose of the list is not to limit the material composition of the nonwoven textile according to the invention but to provide examples of possible polymers. The fibres may be mono-component or multi-component fibres. Amongst multi-component fibres belong, in particular, bi-component fibres such as, for example, fibres with core-and-sheath or side-by-side arrangements.

The individual layers of fibres may be from the same material but may also be made from various materials or their blends. Likewise, the individual layers may consist of the same type of fibres or may contain various layers of various types of fibres within the overall composite. For example it is possible to combine a layer of bi-component fibres with a layer of mono-component fibres or a layer produced essentially from a single material with a layer produced from a blend of polymers and other adjunct materials.

In the hygiene industry, barrier properties are generally understood to mean resistance against the seepage of polar liquids (e.g. water or synthetic urine). For other applications, however, it may be desirable for the barrier to be polar liquid wettable. For such cases, certain naturally hydrophobic materials may be treated using various agents so as to attain hydrophilic properties. The surface treatment may mean the application of a coating formed by a surface-active compound or wetting agent. One such surface-active compound suitable for the treatment of fibres is supplied by Schill & Silacher GmbH, Böblingen, Germany, under the brand name Silastol PHP 90. A person skilled in the art understands that there are various treatment options available in the art and, based on need, can select the correct one for the given application.

Nonwoven textiles may also be treated with other types of surface treatment. One example is a surface treatment comprising of an application of a layer containing an adjunct modifying the surface properties of fibres, specifically reducing surface friction and increasing slipperiness for touch and feel. The preferred surface property modification agents are described for example in patent documentation U.S. 6,632,385 and U.S. 6,803,103 and in the published patent application submitted in the USA under number: 2006/0057921.

Nonwoven textiles may contain layers improving the softness of the overall composite - for example, they may contain compositions described in published patent application WO2014044235.

The "batt" contains individual fibres between which a fixed mutual bond is usually not yet formed even though they may be bonded in certain ways, whilst this pre-bonding may occur during the laying of the layer consisting of loose fibres or shortly thereafter. This pre-bonding, however, still permits a substantial number of the fibres to be freely mobile such that they can be repositioned. Notwithstanding, the "batt" may consist of several layers created by the deposition of fibres from several spinning beams during the spunlaid process. The preferred production method assumes the utilisation of spunmelt (spunlaid) procedures, during which one or several input materials are melted and subsequently under the effect of pressure extruded through spinnerets. Polymer fibres are extruded from the spinnerets. Fibres are then guided through a cooling and extending channel on to a moving belt, and may be deposited in a somewhat random orientation when they fall on the belt, nevertheless, the orientation in the direction of the movement of the nonwoven textile predominates.

Then, the batt can be consolidated using, for example, calendering rollers, which to a certain degree join the fibres by means of bonding points thus forming a structure where the resulting textile has the required mechanical-physical properties, which are considerably affected by the form and extent of bonding.

It is evident that there exist many batt consolidation options - apart from thermal bonding (e.g. calendering or ultrasound bonding), chemical bonding, etc. For the nonwoven textile according to the invention, it is significant that the utilised bonding technology sufficiently joins the individual layers but at the same time does not give rise to "burnthrough", or other significant damage, weakening or even puncturing of the batt in the locations of the bonding points and thereby result in a reduction of its barrier effect.

Nonwoven textiles according to this invention can be used to produce a single or several individual elements of products with absorptive properties. A product with absorptive properties, such as for example, an absorptive hygiene product, containing a layer permeable to liquid, a sorptive core and a barrier layer. The sorptive core may be composed of, for example, a sorptive layer or a sorptive layer containing a superabsorbent. A person skilled in the art will understand that the product with absorptive properties may contain many other elements depending on its purpose. It generally applies that a product with absorptive properties is used in such a manner that the permeable side is oriented towards the approaching liquid. The permeable layer permits the liquid to pass through to the sorptive core, where the liquid is retained. The barrier layer prevents liquid from seeping through in an undesirable direction.

Nonwoven textiles according to this invention may be, during the production of a product with absorptive properties - such as for example absorptive hygiene products, used with advantage to create the external cover layers (backsheet), barrier leg cuffs (BLC) or possibly any other parts of these products, where barrier properties are advantageous.

For disposable absorptive hygiene products, it may be advantageous, when the barrier nonwoven textiles are produced predominantly from polypropylene, for example, containing at least 50%, better yet at least 60%, better yet at least 70%, better yet at least 80%, preferably at least 90% polypropylene. For the use of this type of barrier nonwoven textile in an absorptive hygiene product, it may be particularly advantageous for the total basis weight of the barrier nonwoven textile to be no more than 25 g/m$^2$, better yet no more than 23 g/m$^2$, better yet no more than 20 g/m$^2$, preferably no more than 18 g/m$^2$. Using a lower basis weight nonwoven textile provides both ecological as well as economical advantages. A smaller amount of input raw materials are used, the resulting product is of lighter weight, which brings transport and handling advantages, and finally, during the final disposal of the product a smaller amount of product needs to be processed. Especially advantageous is, when at a lower amount of material the product properties are maintained, without the need to add additives, which may, for example cause problems during disposal of a used product.

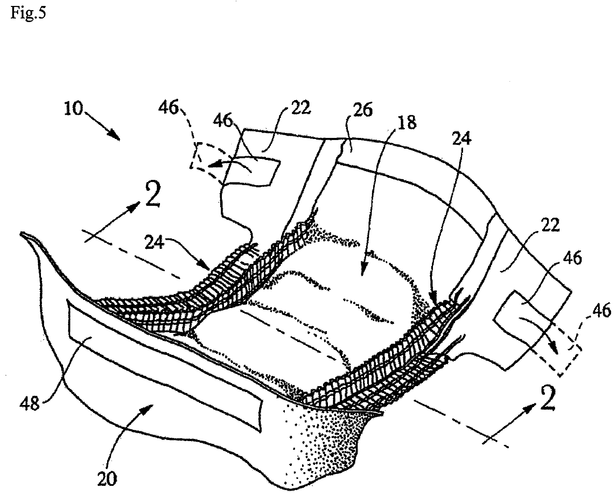

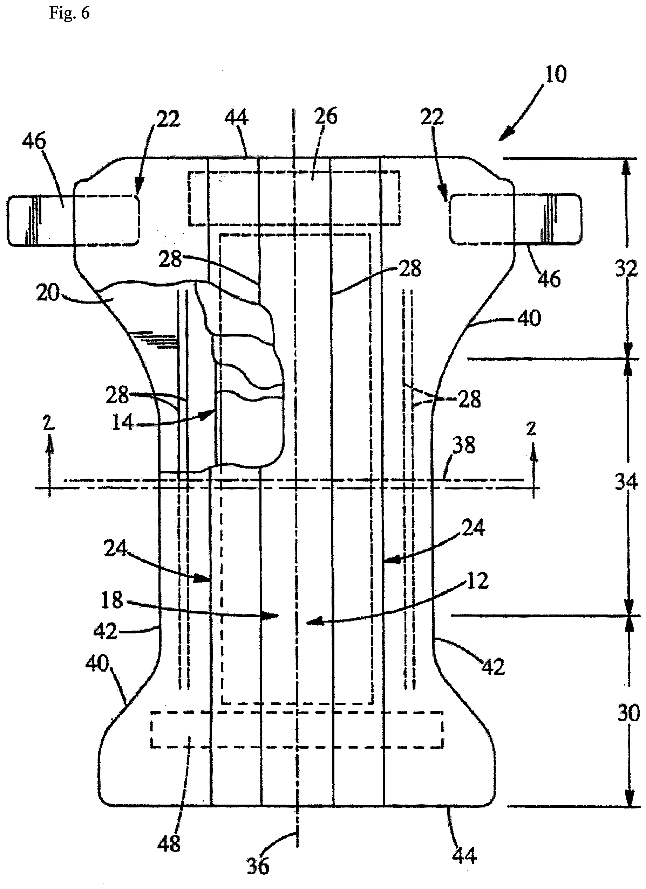

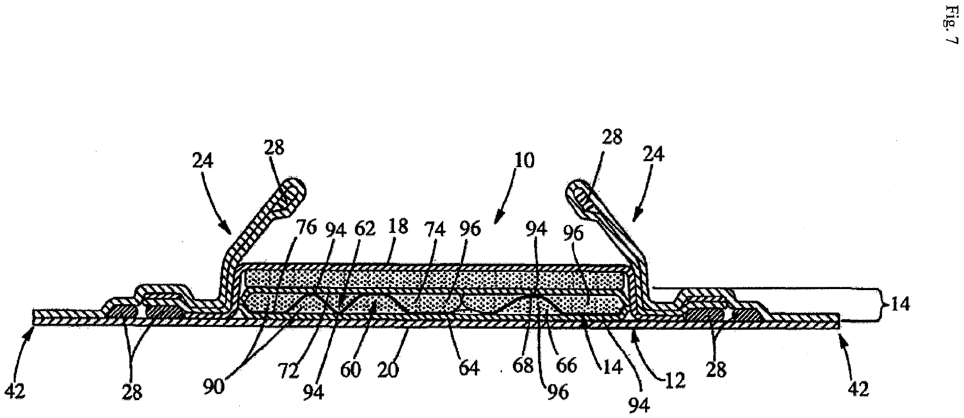

Fig. 5 shows a perspective view of a disposable diaper **10,** which is shown in the loosened, folded out position, in which it may be found when placed on a horizontal surface. Fig. 6. contains a view from above at the same diaper **10,** which is shown in a folded out and flattened state, when it is not pulled tight (i.e. when the effect of its inner tensioning elements are not applying force)), whilst a part of this diaper **10** is cut away in order to make the inner structure visible. In fig. 6, the diaper **10** is shown with the schematic addition of its longitudinal axis **36** and transversal axis **38.** The parts of the diaper **10,** which come into contact with the body of the user are oriented on the image in fig. 5 upwards and on the image in fig. 6 towards the observer. Fig. 7 shows the cross-section of the diaper according to fig. 6, whilst the level of the cut is designated as 2-2 in fig. 6.

The diaper **10** may, in general, contain a coat **12** and an absorptive core **14** arranged inside the coat. The core **12** may at the same time contain also the main body of the diaper **10.**

The coat **12** may, furthermore, also contain an inner surface layer **18,** which may be permeable for liquids, and an outer cover layer **20,** which may on the other hand be impermeable. The absorptive core **14** may be enclosed between the inner surface layer **18** and the outer cover layer **20.** The coat **12** may then, furthermore, contain side flaps **22,** cuffs **24** from an elastic material, which cling to the legs of the user, and an elastic element **26** for pulling tight around the waist. The coat **12** may also contain a fastening system, which may comprise of at least one fastening element **46** and at least one contact area **48.** The inner surface layer 18 and/or the outer cover layer 20 may always also include at least one separation layer created from nonwoven textile, which is described below.

The cuffs **24,** which cling to the legs of the user as well as the elastic element **26** for tightening around the waist, as a rule, include elastic elements **28.** One end part of the diaper **10** may be arranged as the first waist area **30** of the diaper **10.** The opposite part of the diaper **10** may be arranged as the second waist area **32** of the diaper **10.** The middle part of the diaper **10** may be arranged as the crutch area **34,** which is spread out longitudinally between the first and the second waist area **30,** resp. **32.**

In order to be able to wind the diaper **10** around the body of the user, fasten and secure it in the necessary position, the second waist part **32** is fitted with at least one fastening element **46,** by means of which it is attached to the first waist part **30,** to create a closed opening for the legs and an articulated opening for the waist. When the diaper is fastened, the fastening system transfers the load caused by the tensile force oriented around the articulated opening for the waist.

The diaper **10** may be fitted with a fastening system that can be fastened multiple times or alternatively it may be created in the form of diaper pants. On products with absorptive properties produced in individually graduated sizes, the coat **12** and the absorptive core **14** may form the main part of the composite structure of the diaper **10,** to which other elements are added. The inner surface layer **18,** the outer cover layer **20** and the absorptive core **14** may, however, also be arranged in a multitude of known arrangements.

The outer cover layer **20** may be joined with the inner surface layer **18.** The outer cover layer **20** may, at the same time, serve the purpose of preventing the soiling of other external items, which may come into contact with the diaper **10,** such as for example, bed linen and parts of clothing by excretions absorbed by the absorptive core **14** and contained inside the diaper **10.** From the arrangement shown in fig. 2B, it is evident that the outer cover layer **20** may be, essentially, impermeable for fluids (e.g. urine) and may be formed as a multi-layer structure containing a nonwoven textile **21** and a thin polymer film **23,** performed, for example, as a thermoplastic film at a thickness of approximately 0.012 mm (0.5 thousandths of an inch) up to approximately 0.051 mm (2.0 thousandths of an inch). The nonwoven textile **21** may then be the here described nonwoven textile with barrier properties.

In other advantageous configurations, nonwoven textiles according to this invention may be also used for the production of protective garments and other aids in industry and healthcare. Both in industry and in healthcare, protective garments and other aids are categorised into several classes depending on the degree of protection provided. The nonwoven textile according to the invention, for example from polypropylene, may itself be used, for example, for lower classes of protective garments and aids that provide namely resistance against seepage of water or highly polar (namely water-based) solutions. For higher protection classes, where resistance against the seepage of non-polar fluids and their solutions is required, due to its high wettability by this type of solution, it is not suitable to use a nonwoven textile from a non-polar polymer (for example polypropylene). If its use is, for other reasons, desirable, it is necessary to change the surface properties of its fibres by, for example, the use of a suitable additive or surface treatment. For certain applications, where protection against electrostatic charge is required, a maximum permissible surface resistance for the used materials is defined. Here too, the key is a suitable choice of polymers, if for other reasons it is desirable to use, for example, polypropylene, then it is necessary to change the surface properties of its fibres by, for example, the use of a suitable additive or surface treatment. For certain applications a combination of the above described properties is required - typically a minimum water column height together with a minimum resistance against the seepage of non-polar liquids (alcohol repellency) and with a minimum specific surface resistance of the material. It is known in the art how to achieve the required effect by either a pure surface treatment or also a suitable combination of additives and surface treatment.

**Examples**

For example nonwoven textile I. according to the invention (see fig. 3a), for example, contains a barrier layer A consisting of fibres with a smaller diameter. The fibres which form the layer may be produced, for example, using advanced meltblown technology Nanospun MB from REICOFIL - Dietip No.117 fitted on the company's pilot production line. The described technology makes it possible to produce, for example, polypropylene fibres (density of 0.93 g/cm$^3$) with a median diameter of 0.5-2 microns. The layer may also contain a small share of fibres with a significantly greater diameter than the median diameter of the fibres. The minimum basis weight of the layer A is given by a specific median diameter of fibres.

For example, for fibres with a median diameter of 1.3 microns, the minimum basis weight is 0.19 g/m$^2$ (corresponds to 20% TCC), better yet 0.24 g/m$^2$ (corresponds to 25% TCC), preferably 0.28 g/m$^2$ (corresponds to 30% TCC).

The nonwoven textile according to the invention furthermore contains an auxiliary barrier layer B consisting of fibres with a generally higher median diameter of fibres than the layer A, whilst the ratio of the median fibre diameter of the layer A to the layer B expressed using coefficient x, is at least 0.25, preferably 0.3 and concurrently less than or equal to 1.0. Furthermore, the sum of the TCC of the layer A and TCC of the layer B is at least 50%, better yet at least 60%, better yet at least 70%, preferably at least 100%. Fibres forming the layer B may thus be found in the range from 1,625 nm (when x = 0.25) to 2,600 nm (when x = 1).

Fibres forming the layer B may be produced, for example, from polypropylene using the known meltblown-type technology (for example using Reicofil technology). The described technology makes it possible to produce fibres with a median diameter of 1.5-5 microns.

The possible basis weights of layer B are provided in the table below; those for which a different technology than described in the aforementioned example would be required are italicised:

| Layer | Median diameter of fibres in the layer | Minimum g/m² of the observed layer | |
|---|---|---|---|
| **TCC A** | Microns | **20%** | **25%** |
| **A** | 1.300 | 0.19 g/m$^2$ | 0.24 g/m$^2$ |
| **TCC B (= 50%-TCC A)** | | **30%** | **25%** |
| **B, x=0.25** | 1.625 | *0.36 g/m$^2$* | *0.30 g/m$^2$* |
| **B, x=0.3** | 1.690 | *0.37 g/m$^2$* | *0.31 g/m$^2$* |
| **B, x=0.5** | 1.950 | *0.43 g/m$^2$* | *0.36 g/m$^2$* |
| **B, x=1** | 2.600 | *0.57 g/m$^2$* | *0.47 g/m$^2$* |

Furthermore, the nonwoven textile contains two D layers consisting of, for example, polypropylene fibres produced using spunbond technology with a fibre diameter of 1.5-2.5 den, which adjoin to the layer A and B and together form a DABD structure. The layer D has no effect on the principle of the described invention. The layer D may have a basis weight of at least 1 g/m2, better yet 2 g/m2, preferably 3 g/m2 to 30 g/m2, better yet up to 15 g/m2, preferably up to 10 g/m$^2$ .

The nonwoven textile is consolidated, for example, using a thermal calender.

The advantages of the above-described example of the nonwoven textile I. are shown in examples 1-4: SMS-type nonwoven textile with a total basis weight of 17 g/m$^2$, consisting of 14 g/m$^2$ of spunbond-type fibres and 3 g/m$^2$ of meltblown-type fibres, is produced in a continuous production process using three Reicofil 4a-type spunbond spinning beams and two meltblown spinning beams, which are precisely defined in the examples, arranged consecutively in the order S1, S2, M1, M2, S3. A constant belt speed is maintained.

A homopolymer of polypropylene (Tatren HT2511 from Slovnaft Petrochemicals) is dosed into all spunbond spinning beams S1, S2, S3. A person skilled in the art will understand that the specific setup of the production line depends on the specific equipment. The polymer is first melted in the extruder and subsequently brought to the spunbond spinnerets. The created fibres under the spinnerets are pulled off and drawn by an air current with a temperature of 20-35°C. The drawn fibres are collected on a moving belt. In examples 1-4, layer D consists of spunbond layers, therefore it will not be specified later.

A homopolymer of polypropylene (Borflow HL 512 from Borealis) is dosed into each of the meltblown spinning beams. A person skilled in the art will understand that the specific setup of the production line depends on the specific equipment. Differentiation of the meltblown beams is provided in the individual examples. The polymer is first melted in the extruder and subsequently brought to the meltblown spinnerets. The fibres are blown off by a current of air (250-280°C) from underneath the spinnerets and collected on a moving belt. The textile is then consolidated using a thermal calender with a pair of heated rollers, whilst one of the rollers has a projecting emboss pattern. The temperature of the calender rollers (smooth roll/embossed roll) is 150°C/145°C and a pressure of approx. 90 n/mm is applied.

**Example 1 - DBD (comparative example):**

Both used meltblown beams are of the Reicofil type and together they create a homogeneous layer of fibres, which in principle correspond to fibres forming layer B in examples 3+4 according to the invention. See fig. 4-a.

**Example 2 - DAD (comparative example):**

Fibre layer A is formed by a meltblown beam using advanced meltblown technology Nanospun MB from REICOFIL - Dietip No.117 fitted on a pilot line from REICOFIL and forms a layer of fibres, which in principle corresponds to the fibres forming layer A in examples 3+4 based on the invention. See fig. 4-b.

**Example 3: DABD (example according to the invention)**

One of the used beams is a Reicofil type meltblown beam and creates layer B.

The second beam is an advanced meltblown technology Nanospun MB from REICOFIL - Dietip No.117 that is installed on a REICOFIL pilot line, and forms layer A. See fig. 4-c.

The ratio of the basis weight of layer A and layer B is 1:1.

**Example 4: DABD (example according to the invention)**

One of the used beams is a Reicofil 4 type meltblown beam and creates layer B.

The second beam is an advanced meltblown technology Nanospun MB from REICOFIL - Dietip No.117 that is installed on a REICOFIL pilot line, and forms layer A. See fig. 4-c.

The ratio of the basis weight of layer A and layer B is 2:1.

The fibre diameters were measured optically on an electron microscope where a shot of the nonwoven textile was first taken from the side of the observed layer at a suitable resolution and subsequently at least 100 individual fibres were marked and their diameters were measured. For examples 1 and 2, the distribution of the fibre diameters is provided in the following table:

| In examples 3+4 corresponds to layer | Sample | Min (nm) | AVG (nm) | Max (nm) | 25th percentile | 50th percentile = median fibre diameter in the layer | 75th percentile | Number of measured fibres |
|---|---|---|---|---|---|---|---|---|
| B | **Example 1** | 986 | 2,967 | 8,490 | 1,916 | **2,460** | 3,864 | 152 |
| A | **Example 2** | 358 | 1,565 | 5,215 | 890 | **1,367** | 1,881 | 145 |

In the same way, samples of other examples were measured. Hereinafter only median diameters of fibres in the given layers are provided.

The created barrier is assessed using the height of the water column and air permeability - see table:

| Example: | 1 | 2 | 3 | 4 |
|---|---|---|---|---|
| Type of example: | comparative | comparative | according to the invention | according to the invention |
| NT type: | DBD | DAD | DABD | DABD |
| Observed layers: | B | A | AB | AB |
| Total composition of NT: | SSMMS | SSMMS | SSMMS | SSMMS |
| Shown in fig.: | 4-a | 4-b | 4-c | 4-c |
| $g/m^2$ of the observed layers: | 3 | 3 | 1.5+1.5 | A: 2 + B: 1 |
| Total $g/m^2$ | 17 | 17 | 17 | 17 |
| Median of fibre diameters of layer A (nm): | - | 1,367 | 1,320 | 1,356 |
| TCC of layer A (%) | - | 300 % | 156% | 202% |
| Median of fibre diameters in layer B - dBm (nm): | 2,460 | - | 2,390 | 2,478 |
| TCC of layer B (%) | 167 % | - | 86% | 55% |
| Sum of TCC for layers A+B (%) | 167% | 300% | 242% | 257% |
| x = (B-A)/A: | - | - | 0.81 | 0.83 |
| Water column (mm) Fluid = $H_2O$ Fluid applied in the direction DABD | 243 | 314 | 358 | 403 |
| Water column (mm) Modified surface tension of fluid - model "adult urine" Fluid applied in the direction DABD | 196 | 226 | 315 | - |
| Water column (mm) Modified surface tension of fluid - model "child urine" Fluid applied in the direction DABD | 165 | 201 | 273 | - |
| Air permeability ($l/m^2/s$) | 2,468 | 1,254 | 778 | 968 |

From the provided results, it is evident that the barrier capacity (both the water column as well as air permeability) is significantly higher on the nonwoven textile based on the invention (DABD), where the synergistic effect of the combined layers occurs. Contrary to theoretical assumptions, that the best results will be achieved on the thickest layers of finest fibres (DAD). Measurement with a lower surface tension fluid modelling adult urine and child urine shows the same trend, where the nonwoven textile based on the invention (DABD) clearly exhibits the best results.

A different nonwoven textile II. according to the invention (see fig. 3-2), contains a barrier layer A consisting of fibres with a smaller diameter, for example from metallocene polypropylene. The fibres forming the layer were produced using spunbond technology with a production line setup, where the fibres forming Layer A achieved a median fibre diameter of 0.8-1.5 den. The minimum basis weight of layer A is given by a specific median diameter of fibres. For example, for fibres with a median diameter of 1 den (= for PP with a density of 0.93 $g/cm^3$ the corresponding fibre diameter is 13 microns) the minimum basis weight is 1.90 $g/m^2$ (corresponding to 20% TCC), better yet 2.37 $g/m^2$ (corresponding to 25% TCC), preferably 2.85 $g/m^2$ (corresponding to 30% TCC)

The nonwoven textile according to the invention furthermore contains an auxiliary barrier layer B consisting of fibres with a generally higher median diameter of fibres than layer A, whilst the ratio of the median fibre diameter of layer A to layer B expressed using coefficient x, is at least 0.25, preferably 0.3 and concurrently less than or equal to 1.0. Furthermore, the sum of the TCC of the layer A and TCC of the layer B is at least 50%, better yet at least 60%, better yet at least 70%, preferably at least 100%. Fibres forming the layer B may thus be found in the range from 16.25 microns (when x = 0.25) to 26 microns (when x = 1).

Fibres forming the layer B may be produced, for example, from polypropylene using the known spunbond-type technology (for example using Reicofil 4 technology). The described technology makes it possible to produce fibres with a median diameter of 1.6 - 3 den (for the specified PP corresponding to 15.7 - 21.5 microns).

The possible basis weights of the layer B are provided in the table below; those for which a different technology than described in the aforementioned example would be required are italicised:

| Layer | Median diameter of fibres in the layer | Minimum g/m$^2$ of the observed layer | |
|---|---|---|---|
| TCC A | Microns | 20% | 25% |
| A | 13 | 1.90 g/m$^2$ | 2.37 g/m$^2$ |
| TCC B (= 50%-TCC A) | | 30% | 25% |
| B, x=0.25 | 16 | 3.56 g/m$^2$ | 2.97 g/m$^2$ |
| B, x=0.3 | 17 | 3.70 g/m$^2$ | 3.09 g/m$^2$ |
| B, x=0.5 | 20 | 4.27 g/m$^2$ | 3.56 g/m$^2$ |
| B, x=1 | 26 | 5.70 g/m$^2$ | 4.75 g/m$^2$ |

Furthermore, the nonwoven textile contains layer D1 consisting of, for example, polypropylene fibres produced using meltblown technology with a fibre diameter of 2-5 microns, which adjoin to layer A and together form a DABD structure. Layer D1 has no effect on the principle of the described invention. Layer D1 may have a basis weight from at least 1 g/m2, better yet 2 g/m2, preferably 3 g/m2 to 30 g/m$^2$, better yet up to 15 g/m2, preferably up to 10 g/m$^2$ .

Furthermore, the nonwoven textile contains another layer D2 consisting of, for example, polypropylene fibres produced using spunbond technology (for example using Reicofil 4 technology), which adjoin the layer D1. Together they form the structure BAD1D2. The described technology makes it possible to produce fibres with a median diameter of 1.6 - 3 den (for the specified PP corresponding to 15.7 - 21.5 microns). The layer D2 has no effect on the principle of the described invention. The layer D2 may have a basis weight of at least 1 g/m2, better yet 2 g/m2, preferably 3 g/m2 to 30 g/m$^2$, better yet up to 15 g/m2, preferably up to 10 g/m$^2$ .

The nonwoven textile is consolidated, for example, using a thermal calender.

The advantages of the above-described example of nonwoven textile II. are shown in examples 5-6: SMS-type nonwoven textile with a total basis weight of 15 g/m$^2$, consisting of 12 g/m$^2$ of spunbond-type fibres and 3 g/m$^2$ of meltblown-type fibres, is produced in a continuous production process using three Reicofil 4a-type spunbond beams and two meltblown beams, which are precisely defined in the examples, arranged consecutively in the order S1, S2, M1, M2, S3. A constant belt speed of 750 m/min. is maintained.

A homopolymer of polypropylene (defined in detail in individual cases) is dosed into all spunbond beams S1, S2, S3. A person skilled in the art will understand that the specific setup of the production line depends on the specific equipment. The polymer is first melted in the extruder and subsequently brought to the spunbond spinnerets. The created fibres under the spinnerets are pulled off and drawn by an air current with a temperature of 20-35°C. The drawn fibres are collected on a moving belt. Beam S3 forms layer D2.

A homopolymer of polypropylene (Borflow HL 512 from Borealis) is dosed into each of the meltblown beams. A person skilled in the art will understand that the specific setup of the production line depends on the specific equipment. The polymer is first melted in the extruder and subsequently brought to the meltblown spinnerets. The fibres are blown off by a current of air (250-280°C) from underneath the spinnerets and collected on a moving belt. Fibres produced in this way achieve a median fibre diameter of approx. 2-5 microns. In examples 5-6, layer D1 consists of meltblown layers, therefore it will not be specified later.

The textile is then consolidated using a thermal calender with a pair of heated rollers, whilst one of the rollers has a projecting emboss pattern. The temperature of the calender rollers (smooth roll/embossed roll) is 150°C/145°C and a pressure of approx. 90 n/mm is applied.

**Example 5 - BD1D2 (comparative example):**

All three REICOFIL 4 spunbond beams dose the same polymer (Tatren HT2511 from Slovnaft Petrochemicals) at the same setting and output parameters. Beams S1 and S2 together create a homogeneous layer of fibres, which in principle correspond to fibres forming the layer B in example 6. Beam S3 forms layer D2. See Fig. 4-d.

**Example 6 - BAD1D2 (example according to the invention):**

All three spunbond beams are type REICOFIL 4.

The first (S1) and third (S3) spunbond beams dose the same polypropylene homopolymer (Tatren HT2511 from Slovnaft Petrochemicals) at the same setting and output parameters. The first beam produces the layer B, the third beam produces the layer D2.

The second spunbond beam (S2) doses a metallocene-type polypropylene homopolymer (MR 2001 from Total) at a lower output than the first and the third beam, so that the resulting fibre thickness is approximately 1 denier, this beam creates layer A. See fig. 4-e.

The created barrier is assessed using the height of the water column and air permeability - see table:

| | Example: | 5 | 6 |
|---|---|---|---|
| | Type of example: | comparative | according to the invention |
| | NT type: | BD1D2 | BAD1D2 |
| | Observed layers: | B | BA |
| | Total composition of NT: | SSMMS | SSMMS |
| | Shown in fig.: | 4-d | 4-e |
| | $g/m^2$ of the observed layers: | 9.3 | B: 4.67+ A: 3.33 |
| | Total $g/m^2$ | 15 | 15 |
| | Median diameter of fibres of layer A - dAm (nm): | - | 12,920 (1.08 den) |
| | TCC of layer A (%) | - | 35% |
| | Median diameter of fibres of layer B - dBm (nm): | 17,300 (1.95 den) | 17,320 (1.95 den) |
| | TCC of layer B (%) | 74% | 37% |
| | Sum of TCC for layers A+B (%) | 74% | 72% |
| | x = (B-A)/A: | - | 0.34 |
| | Water column (mm) Fluid = $H_2O$ Fluid applied in the direction of DDBA | 264 | 291 |
| | Air permeability $(l/m^2/s)$ | 1,042 | 854 |

A different nonwoven textile III. according to the invention (see fig. 3-1), for example, contains a barrier layer A consisting of fibres with a smaller diameter. The fibres that form the layer may be produced, for example, using advanced meltblown technology Nanospun MB from REICOFIL - Dietip No.117 fitted on the company's pilot production line. The described technology makes it possible to produce, for example, polypropylene fibres (density of 0.93 $g/cm^3$) with a median diameter of 0.5-2 microns. The layer may also contain a small share of fibres with a significantly greater diameter than the median diameter of the fibres. The minimum basis weight of the layer A is given by a specific median diameter of fibres. For example, for fibres with a median diameter of 1.4 microns, the minimum basis weight is 0.20 $g/m^2$ (corresponds to 20% TCC), better yet 0.26 $g/m^2$ (corresponds to 25% TCC), preferably 0.31 $g/m^2$ (corresponds to 30% TCC).

The nonwoven textile according to the invention furthermore contains an auxiliary barrier layer B consisting of fibres with a generally higher median diameter of fibres than layer A, whilst the ratio of the median fibre diameter of the layer A to that of the layer B expressed using coefficient x, is at least 0.25, preferably 0.3 and concurrently less than or equal to 1.0. Furthermore, the sum of the TCC of the layer A and TCC of the layer B is at least 50%, better yet at least 60%, better yet at least 70%, preferably at least 100%. Fibres forming the layer B may thus be found in the range from 1,750 nm (when x = 0.25) to 2800 nm (when x = 1).

Fibres forming the layer B may be produced, for example, from polypropylene using the known meltblown-type technology (for example using Reicofil technology). The described technology makes it possible to produce fibres with a median diameter of 1.5-5 microns.

The possible basis weights of layer B are provided in the table below; those for which a different technology than described in the aforementioned example would be required are italicised:

| Layer | Median diameter of fibres in the layer | Minimum $g/m^2$ of the observed layer | |
|---|---|---|---|
| | | 20% | 25% |
| TCC A | Microns | | |

(continued)

| Layer | Median diameter of fibres in the layer | Minimum g/m² of the observed layer | |
|---|---|---|---|
| A | 1.1 | 0.20 g/m² | 0.26 g/m² |
| TCC B (= 50%-TCC A) | | 30% | 25% |
| B, x=0.25 | 1.75 | 0.38 g/m² | 0.32 g/m² |
| B, x=0.3 | 1.82 | 0.40 g/m² | 0.33 g/m² |
| B, x=0.5 | 2.1 | 0.46 g/m² | 0.38 g/m² |
| B, x=1 | 2.8 | 0.61 g/m² | 0.51 g/m² |

Furthermore, the nonwoven textile contains two D layers consisting of, for example, polypropylene fibres produced using spunbond technology with a fibre diameter of 1.5-2.5 den, which adjoin the layers A and B and together form a DABD structure. The layer D has no effect on the principle of the described invention. The layer D may have a basis weight of at least 1 g/m2, better yet 2 g/m², preferably 3 g/m² to 30 g/m², better yet up to 15 g/m², preferably up to 10 g/m².

The nonwoven textile is consolidated, for example, using a thermal calender.

The advantages of the above-described example of the nonwoven textile III. are shown in examples 7-11: SMS-type nonwoven textile with a total basis weight of 17 g/m², consisting of 14 g/m² of spunbond-type fibres and 3 g/m² of meltblown-type fibres, is produced in a continuous production process using three Reicofil 4a-type spunbond beams and two meltblown beams, which are precisely defined in the examples, arranged consecutively in the order S1, M1, M2, S3. A constant belt speed is maintained.

A homopolymer of polypropylene (Tatren HT2511 from Slovnaft Petrochemicals) is dosed into all spunbond beams S1 and S3. A person skilled in the art will understand that the specific setup of the production line depends on the specific equipment. The polymer is first melted in the extruder and subsequently brought to the spunbond spinnerets. The created fibres under the spinnerets are pulled off and drawn by an air current with a temperature of 20-35°C. The drawn fibres are collected on a moving belt. In examples 7-11, layer D consists of spunbond layers, therefore it will not be specified later.

A homopolymer of polypropylene (Borflow HL 512 from Borealis) is dosed into each of the meltblown beams. A person skilled in the art will understand that the specific setup of the production line depends on the specific equipment. Differentiation of the meltblown beams is provided in the individual examples. The polymer is first melted in the extruder and subsequently brought to the meltblown spinnerets. The fibres are blown off by a current of air (250-280°C) from underneath the spinnerets and collected on a moving belt. The textile is then consolidated using a thermal calender with a pair of heated rollers, whilst one of the rollers has a projecting emboss pattern. The temperature of the calender rollers (smooth roll/embossed roll) is 150°C/145°C and a pressure of approx. 90 n/mm is applied.

**Example 7** - **DBD (comparative example):**

Both used meltblown beams are type Reicofil and together create a homogeneous layer of fibres, which in principle correspond to fibres forming the layer B in examples 9-11 according to the invention. See Fig. 4-f.

**Example 8** - **DAD (comparative example):**

Fibre layer A is formed by a meltblown beam using advanced meltblown technology Nanospun MB from REICOFIL - Dietip No.117 fitted on a pilot line from REICOFIL and forms a layer of fibres, which in principle corresponds to the fibres forming the layer A in examples 9-11 based on the invention. See Fig. 4-g.

**Example 9: DABD (example according to the invention)**

One of the used beams is a Reicofil 4 type meltblown beam and creates the layer B.

The second beam is an advanced meltblown technology Nanospun MB from REICOFIL - Dietip No. 117 that is installed on a REICOFIL pilot line, and forms the layer A.

The ratio of the basis weight of the layer A and the layer B is 2:1.

**Example 10: DABD (example according to the invention)**

One of the used beams is a Reicofil 4 type meltblown beam and creates layer B.
The second beam is an advanced meltblown technology Nanospun MB from REICOFIL - Dietip No. 117 that is installed on a REICOFIL pilot line, and forms the layer A.
The ratio of the basis weight of the layer A and the layer B is 1:1.

**Example 11: DABD (example according to the invention)**

One of the used beams is a Reicofil 4 type meltblown beam and creates the layer B.
The second beam is an advanced meltblown technology Nanospun MB from REICOFIL - Dietip No. 117 that is installed on a REICOFIL pilot line, and forms the layer A.
The ratio of the basis weight of the layer A and the layer B is 2.1:0.9.

| Example: | 7 | 8 | 9 | 10 | 11 |
|---|---|---|---|---|---|
| Type of example: | comparative | comparative | according to the invention | according to the invention | according to the invention |
| NT type: | DBD | DAD | DABD | DABD | DABD |
| Layers based on the invention: | B | A | AB | AB | AB |
| Total composition of NT: | SMMS | SMMS | SMMS | SMMS | SMMS |
| Shown in fig.: | 4-f | 4-g | 4-h | 4-h | 4-h |
| $g/m^2$ layers based on the invention: | 3 | 3 | B: 1.0 + A: 2.0 | 1.5+1.5 | B: 0,9 + A: 2.1 |
| Total $g/m^2$ | 17 | 17 | 17 | 17 | 17 |
| Median diameter of fibres of **layer** A (nm): | - | 1420 | 1430 | 1520 | 1400 |
| TCC of **layer** A (%) | - | 289% | 191% | 135% | 205% |
| Median diameter of fibres of layer B (nm): | 2330 | - | 2450 | 2200 | 2510 |
| TCC of **layer** B (%) | 176% | - | 56% | 93% | 49% |
| Sum of TCC for layers A+B (%) | 176% | 289% | 247% | 228% | 254% |
| x = (B-A)/A: | - | - | 0.71 | 0.45 | 0.79 |
| Water column (mm) Fluid = $H_2O$ Fluid applied in the direction DABD | 201.5 | 295 | 378.5 | 401.5 | 416 |

From the provided results, it is evident that the barrier capacity (water column) is significantly higher for the nonwoven textile based on the invention (DABD), where the synergistic effect of the combined layers occurs. Contrary to theoretical assumptions, that the best results will be achieved on the thickest layers of finest fibres (DAD).
Nonwoven textile IV. according to the invention (see fig. 3-1), for example, contains a barrier layer A consisting of fibres with a smaller diameter. The fibres that form the layer may be produced, for example, using advanced meltblown technology Nanospun MB from REICOFIL - Dietip No.118 fitted on the company's pilot production line. The described technology makes it possible to produce, for example, polypropylene fibres (density of 0.93 $g/cm^3$) with a median diameter of 0.4-1.5 microns. The layer may also contain a small share of fibres with a significantly greater diameter than the median diameter of the fibres. The minimum basis weight of layer A is given by a specific median diameter of fibres. For example, for fibres with a median diameter of 1.0 micron, the minimum basis weight is 0.15 $g/m^2$ (corresponds to 20% TCC), better yet 0.18 $g/m^2$ (corresponds to 25% TCC), preferably 0.22 $g/m^2$ (corresponds to 30% TCC).
The nonwoven textile according to the invention furthermore contains an auxiliary barrier layer B consisting of fibres with a generally higher median diameter of fibres than layer A, whilst the ratio of the median fibre diameter of the layer A to that of the layer B expressed using coefficient x, is at least 0.25, preferably 0.3 and concurrently less than or equal to 1.0. Furthermore, the sum of the TCC of layer A and TCC of layer B is at least 50%, better yet at least 60%, better yet at least 70%, preferably at least 100%. Fibres forming the layer B may thus be found in the range from 1,250 nm (when x = 0.25) to 2,000 nm (when x = 1).

The fibres that form the layer B may be produced, for example, from polypropylene using advanced meltblown technology Nanospun MB from REICOFIL - Dietip No.117 fitted on the company's pilot production line. The described technology makes it possible to produce, for example, polypropylene fibres (density of 0.93 $g/cm^3$) with a median diameter of 0.5-2.0 microns. The possible basis weights of the layer B are provided in the table below; those for which a different technology than described in the aforementioned example would be required are italicised:

| Layer | Median diameter of fibres in the layer | Minimum $g/m^2$ of the observed layer | |
|---|---|---|---|
| TCC A | Microns | 20% | 25% |
| A | 1.0 | 0.15 $g/m^2$ | 0.18 $g/m^2$ |
| TCC B (= 50%-TCC A) | | 30% | 25% |
| B, x=0.25 | 1.250 | 0.27 $g/m^2$ | 0.23 $g/m^2$ |
| B, x=0.3 | 1.300 | 0.28 $g/m^2$ | 0.24 $g/m^2$ |
| B, x=0.5 | 1.500 | 0.33 $g/m^2$ | 0.27 $g/m^2$ |
| B, x=1 | 2.000 | 0.44 $g/m^2$ | 0.37 $g/m^2$ |

Furthermore, the nonwoven textile contains two D layers consisting of, for example, polypropylene fibres produced using spunbond technology with a fibre diameter of 1.5-2.5 den, which adjoin to layers A and B and together form a DABD structure. Layer D has no effect on the principle of the described invention. Layer D may have a basis weight of at least 1 g/m2, better yet 2 g/m2, preferably 3 $g/m^2$ to 30 g/m2, better yet up to 15 g/m2, preferably up to 10 $g/m^2$ .

The nonwoven textile is consolidated, for example, using a thermal calender.

The advantages of the above-described example of nonwoven textile IV. are shown in examples 12-13.

SMS-type nonwoven textile with a total basis weight of 17 $g/m^2$, consisting of 14 $g/m^2$ of spunbond-type fibres and 3 $g/m^2$ of meltblown-type fibres, is produced in a continuous production process using three Reicofil 4a-type spunbond beams and meltblown beams, which are precisely defined in the examples, arranged consecutively in the order S1, M1, M2, S3. A constant belt speed is maintained.

A homopolymer of polypropylene (Tatren HT2511 from Slovnaft Petrochemicals) is dosed into all spunbond beams S1, S3. A person skilled in the art will understand that the specific setup of the production line depends on the specific equipment. The polymer is first melted in the extruder and subsequently brought to the spunbond spinnerets. The created fibres under the spinnerets are pulled off and drawn by an air current with a temperature of 20-35°C. The drawn fibres are collected on a moving belt. In examples 12-13, layer D consists of spunbond layers, therefore it will not be specified later.

A homopolymer of polypropylene (Borflow HL 512 from Borealis) is dosed into each of the meltblown beams. A person skilled in the art will understand that the specific setup of the production line depends on the specific equipment. Differentiation of the meltblown beams is provided in the individual examples. The polymer is first melted in the extruder and subsequently brought to the meltblown spinnerets. The fibres are blown off by a current of air (250-280°C) from underneath the spinnerets and collected on a moving belt. The textile is then consolidated using a thermal calender with a pair of heated rollers, whilst one of the rollers has a projecting emboss pattern. The temperature of the calender rollers (smooth roll/embossed roll) is 150°C/145°C and a pressure of approx. 90 n/mm is applied.

**Example 12: DABD (example according to the invention)**

One of the used beams is an advanced meltblown technology Nanospun MB from REICOFIL - Dietip No.117 that is installed on a REICOFIL pilot line, and forms layer B. The second beam used is an advanced meltblown technology Nanospun MB from REICOFIL - Dietip No.118 that is installed on a REICOFIL pilot line, and forms layer A. See Fig. 4-h.

The ratio of the basis weight of layer A and layer B is 1:2.

**Example 13: DABD (example according to the invention)**

One of the used beams is an advanced meltblown technology Nanospun MB from REICOFIL - Dietip No.117 that is installed on a REICOFIL pilot line, and forms layer B. The second beam used is an advanced meltblown technology Nanospun MB from REICOFIL - Dietip No.118 that is installed on a REICOFIL pilot line, and forms the layer A. See Fig. 4-h.

The ratio of the basis weight of the layer A and the layer B is 1:1.

The fibre diameters were measured optically on an electron microscope where a shot of the nonwoven textile was first taken at a suitable resolution and subsequently at least 100 individual fibres were marked and their diameters were measured.

The created barrier is assessed using the height of the water column - see table:

| Example: | 12 | 13 |
|---|---|---|
| Type of example: | according to the invention | according to the invention |
| NT type: | DABD | DABD |
| Layers based on the invention: | AB | AB |
| Total composition of NT: | SMMS | SMMS |
| Shown in fig.: | 4-h | 4-h |
| g/m$^2$ layers based on the invention: | A: 1.0 + B: 2.0 | A: 1.5 + B: 1.5 |
| Total g/m$^2$ | 17 | 17 |
| Median diameter of fibres of **layer** A (nm): | 1050 | 1090 |
| TCC of layer A (%) | 130% | 188% |
| Median diameter of fibres of layer B (nm): | 1430 | 1370 |
| TCC of layer B (%) | 191% | 150% |
| Sum of TCC for layers A+B (%) | 321% | 338% |
| x = (B-A)/A: | 0.36 | 0.26 |
| Water column (mm) | 385 | 394.1 |
| Fluid = H$_2$0 Fluid applied in the direction DABD | | |

Absorptive hygiene product - diaper 10 containing a coat 12 and an absorptive core 14 arranged inside the coat. The coat 12, furthermore, contains an inner surface layer **18,** permeable for fluids, and an outer cover layer 20, impermeable for fluids. The absorptive core 14 is enclosed between the inner surface layer 18 and the outer cover layer 20. The coat 12, furthermore, contains side flaps 22, cuffs 24, which cling to the legs of the user, and an elastic element 26 for pulling tight around the waist. The coat 12 also includes a fastening system.

**Example 14** - **NT applications according to the invention** - **backsheet laminate**

The outer cover layer 12 of the "absorptive hygiene product - diaper" is formed by a laminate containing a vapour permeable polypropylene film and a barrier nonwoven textile described in example 10. The total basis weight of the outer cover layer 12 is 30 g/m$^2$.

**Example 15** - **NT applications according to the invention** - **backsheet without lamination**

The outer cover layer 12 of the "absorptive hygiene product - diaper" is formed by a barrier nonwoven textile described in example 11.

**Example 16** - **NT applications according to the invention** - **BLC without lamination**

The cuffs 24 of the "absorptive hygiene product - diaper" are created by a combination of a barrier nonwoven textile described in example 11 and an elastic element ensuring that the cuff clings to the legs of the user.

Nonwoven textile V. according to the invention is produced in the same way as nonwoven textile I., with the following differences:

- 3 meltblown beams are used, whilst 2 are located directly next to each other to create layer B and one beam creates layer A.
- the total basis weight is 34 g/m$^2$, where 26 g/m$^2$ is comprised of spunbond fibres and 8 g/m$^2$ of meltblown-type fibres.

- a constant belt speed of 340 m/min. is maintained.
- the temperature of the calender rollers (smooth roll/embossed roll) is 133°C/136°C and a pressure of approx. 80 N/mm is applied.

Advantages are shown in examples 17-18.

### Example 17: DABD (example according to the invention)

One type of the used beams is a Reicofil 4 type meltblown beam and creates layer B.
The second beam is an advanced meltblown technology Nanospun MB from REICOFIL - Dietip No.117 that is installed on a REICOFIL pilot line, and forms layer A.
The ratio of the basis weight of layer A and layer B is 3:5.

### Example 18 - DABD (example according to the invention) + Lurol

One type of the used beams is a Reicofil 4 type meltblown beam and creates layer B.
The second beam is an advanced meltblown technology Nanospun MB from REICOFIL - Dietip No.117 that is installed on a REICOFIL pilot line, and forms layer A.
The ratio of the basis weight of layer A and layer B is 3:5.
The resulting nonwoven textile was impregnated by a Water-based solution Lurol ASY, Goulston Technologies; with a concentration of 5 % using an insert dip roller (kiss roll) and dried on a drum dryer. The material was, subsequently, stored in a conditioned warehouse for a period of 5 days, where the temperature remained in the range 10 - 30 °C and air humidity did not fall below 60 %.

| Example: | 17 | 18 |
|---|---|---|
| Type of example: | according to the invention | according to the invention |
| NT type: | DABD | DABD |
| Layers based on the invention: | AB | AB |
| Total composition of NT: | SSMMMS | SSMMMS |
| Shown in fig.: | 4-c | 4-c |
| g/m$^2$ layers based on the invention: | A: 3.0 + B: 5.0 | A: 3.0 + B: 5.0 |
| total g/m$^2$ | 34 | 34 |
| Median diameter of fibres of layer A (nm): | 1080 | 1080 |
| TCC of **layer** A (%) | 634% | 634% |
| Median diameter of fibres of **layer** B (nm): | 1492 | 1492 |
| TCC of layer B (%) | 459% | 459% |
| Sum of TCC for layers A+B (%) | 1093% | 1093% |
| x = (B-A)/A: | 0.38 | 0.38 |
| Water column (mm) Fluid = H$_2$0 Fluid applied in the direction DABD | 484 | 453 |
| Air permeability (l/m$^2$/s) | 311 | 301 |
| Specific surface resistance value according to EN 1149 ($\Omega$/m$^2$) | 8.1e13 | 2.8e9 |

### Example 18 - NT application according to the invention - protective garment without treatment and without lamination

Disposable protective garment - overalls made from nonwoven textile described in example 17.

Example 19 - NT application according to the invention - protective garment AS without lamination

Disposable protective garment - pants made from nonwoven textile described in example 18.

Example 20 - NT application according to the invention - protective garment AS with lamination

Disposable protective garment - a coat made from laminate containing vapour permeable polypropylene film and barrier nonwoven textile with antistatic properties described in example 18. Total basis weight of the laminate is 50 g/m$^2$.

Example 21 - NT applications according to the invention - surgical cover sheet

Disposable protective element - surgical cover sheet - protection made from nonwoven textile described in example 17.

Example 22 - NT applications according to the invention - surgical cover sheet with lamination

Disposable protective element - surgical cover sheet - patient cover sheet made from laminate containing vapour permeable polypropylene film and barrier nonwoven textile described in example 17. Total basis weight of the laminate is 55 g/m$^2$.

Nonwoven textile VI. according to the invention is produced in the same way as nonwoven textile I., with the following differences:

- 3 meltblown beams are used, whilst 2 are located directly next to each other to create layer B and one beam creates layer A.
- the total basis weight is 35 g/m$^2$, where 27 g/m$^2$ is comprised of spunbond fibres and 8 g/m$^2$ of meltblown-type fibres.
- a constant belt speed of 340 m/min. is maintained.
- each spunbond beam S1, S2, S3 is dosed with a blend of homopolymer polypropylene (Mosten NB 425) and a functional additive (Hydrepel A 204, Goulston Technologies; in PP masterbatch with a melt flow index of 35 MFI).
- each meltblown beam is dosed with a blend of homopolymer polypropylene (Borflow HL 712 from Borealis) and a functional additive (Hydrepel A 204, Goulston Technologies; in PP masterbatch with a melt flow index of 500 MFI).
- the temperature of the calender rollers (smooth roll/embossed roll) is 133°C/136°C and a pressure of approx. 80 N/mm is applied.

Advantages are shown in examples 23-24.

Example 23: DABD (example according to the invention) - AR

One of the used beams is a Reicofil 4 type meltblown beam and creates layer B.
The second beam is an advanced meltblown technology Nanospun MB from REICOFIL - Dietip No.117 that is installed on a REICOFIL pilot line, and forms layer A.
The ratio of the basis weight of layer A and layer B is 3:5.

Example 24: DABD (example according to the invention) + ASAR

One of the used beams is a Reicofil 4 type meltblown beam and creates layer B.
The second beam is an advanced meltblown technology Nanospun MB from REICOFIL - Dietip No.117 that is installed on a REICOFIL pilot line, and forms layer A.
The ratio of the basis weight of layer A and layer B is 3:5.
The resulting nonwoven textile was impregnated by a water-based solution Lurol ASY, Goulston Technologies; with a concentration of 5 % using an insert dip roller (kiss roll) and dried on a drum dryer. The material was, subsequently, stored in a conditioned warehouse for a period of 5 days, where the temperature remained in the range 10 - 30 °C and air humidity did not fall below 60 %. The delay between the formation of the fibres and the application of the surfactant was less than 1 minute.

| Example: | 23 | 24 |
|---|---|---|
| Type of example: | according to the invention | according to the invention |

(continued)

| Example: | 23 | 24 |
|---|---|---|
| NT type: | DABD | DABD |
| Layers based on the invention: | AB | AB |
| Total composition of NT: | SSMMMS | SSMMMS |
| Shown in fig.: | 4-c | 4-c |
| g/m$^2$ layers based on the invention: | A: 3.0 + B: 5.0 | A: 3.0 + B: 5.0 |
| Total g/m$^2$ | 35 | 35 |
| Median diameter of fibres | 1241 | 1241 |
| Example: | 23 | 24 |
| of layer A (nm): | | |
| TCC of **layer** A (%) | 331% | 331% |
| Median diameter of fibres of **layer** B (nm): | 1902 | 1902 |
| TCC of layer B (%) | 360% | 360% |
| Sum of TCC for layers A+B (%) | 691% | 691% |
| x = (B-A)/A: | 0.53 | 0.53 |
| Water column (mm) Fluid = H$_2$0 Fluid applied in the direction DABD | 514 | 499 |
| Air permeability (l/m$^2$/s) | 336 | 315 |
| Specific surface resistance value according to EN 1149 ($\Omega$/m$^2$) | 9.4e14 | 2.8e9 |
| Alcohol resistance according to EN 1149 ($\Omega$/m$^2$) | 9.8 | 9.5 |

**Example 25** - **NT application according to the invention** - **protective garment AR**

Disposable protective garment - a coat made from nonwoven textile described in example 17.

**Example 26** - **NT application according to the invention** - **protective garment ASAR**

Disposable protective garment - an apron made from nonwoven textile described in example 18.

Testing methods

**Basis weight (g/m$^2$)** is measured on a nonwoven textile using standardised testing methodology EN ISO 9073-1:1989 (corresponding to norm WSP 130.1). For measurement, 10 layers of nonwoven textile are used, sample size is 10 x 10 cm$^2$.

**The basis weight of the** individual layers is in the case where one is knowledgeable of production line configuration a known unit. In the event of an unknown sample, the basis weight of the layers can be approximately determined using various methods. A person skilled in the art is able to select a suitable methodology for specific cases.

For example, it is possible to mechanically separate the nonwoven textile layers from each other and then measure the basis weight as described above.

For example, the optical method can be used to determine in cross section the approximate borders of the individual layers and their fibre packing density. Together with the knowledge of the density of the polymer used, it is then possible to calculate an indicative basis weight of a layer.

The **"Water column (mm)"** is measured on nonwoven textiles using the standardised testing methodology WSP 080.6.R4 (12) issued by the European Disposables and Nonwovens Association (EDANA). A 100 cm$^2$ head is used with a fluid pressure increase rate of 10 mm water column/minute. Unless indicated otherwise, clean water was used for the measurement. To achieve comparable results, it is required that the material is subjected to the water column always from the side of layer A - i.e. AB, DAB, DABDD, DDAB etc. If it is not possible to identify the correct side prior to measurement, it is necessary to measure the sample from both sides and to use the better result for evaluation.

**Permeability of the nonwoven textile** (l/m$^2$/s) is measured on nonwoven textiles using the standardised testing methodology WSP 70.1. issued by the European Disposables and Nonwovens Association (EDANA). A 20 cm$^2$ head at a pressure of 200 Pa is used.

**Specific surface resistance** ($\Omega$/m$^2$) is measured on a nonwoven textile using the standardised testing methodology EN 1149.

**Alcohol resistance (on a scale of 1-10)** is measured on a nonwoven textile using the standardised testing methodology WSP 80.8-2005.

**Median fibre diameter in a layer** is expressed in SI units - micrometers ($\mu$m) or nanometres (nm).

In each sample, it is necessary to determine the individual fibre layers A and B.

In the case where one is knowledgeable of production process configuration it is possible to determine the possible layers by estimation and to verified this by measurement (e.g. during production of an SMS nonwoven textile on a production line with a beam configuration of S1S2M1M2S3, the layers A, B may be either in SB layers - by measurement of layer created by beams S1 and S2 and/or in MB layers - by measurement of the layer created by beams M1 and M2).

When examining an unknown sample, it is appropriate to perform, for example, utilising suitable technology and procedure, a cross-section of the nonwoven textile and in the first indicative measurement to determine whether the composition of the fibres corresponds to a layered textile structure and identify the location of the key layers in the structure. (e.g. in a cross-section of an unknown sample it is possible to compare 2 layers of spunbond fibres, the diameters of which are statistically distributed along the entire surfaces of the section and the layer of meltblown fibres, the diameters of which are distributed in such a way that adjacent to the SB fibres they are rather thicker fibres and in the middle of the layer rather thinner fibres. This leads to the hypothesis that the MB layer is in actual fact formed of three layers M1 / M2 / M3, where it could have the structure BAB. This hypothesis needs to be verified by further measurement).

To determine the median, it is necessary to take a sample of nonwoven textile from at least three locations at least 5 cm away from each other. In each sample, it is necessary to measure the diameter of at least 50 individual fibres for each observed layer. It is possible to use, for example, an optical or electronic microscope (depending on the diameter of the measured fibres). In the event that the diameter of fibres in one sample varies significantly from the other two, it is necessary to discard the entire sample and to prepare a new one. The measured values for each layer composed of all three samples are consolidated into a single set of values from which the median is subsequently determined. It applies that at least 50% of the fibres have a diameter less than or equal to the value of the median and at least 50% of the fibres have a diameter greater than or equal to the median. To identify the median of the given sample set of values, it is sufficient to arrange the values according to size and to take the value found in the middle of the list. In the event that the sample set has an even number of items, usually the median is determined as the arithmetic mean of the values in locations N/2 and N/2+1.

**Claims**

1. Nonwoven textile with barrier properties containing

    a. a first barrier layer A with a theoretical cover coefficient TCC A, and consisting of fibres having a median fibre diameter dAm within the layer; and
    b. a second barrier layer B with a theoretical cover coefficient TCC B, and consisting of fibres having a median fibre diameter dBm in the layer;
    c. a third layer D;

    **wherein**

    i. the first barrier layer A and the second barrier layer B are in direct contact with each other;
    ii. the median fiber diameters within the first and the second layer have a theoretical fibre diameter coefficient x = (dBm-dAm)/dAm, where the theoretical fibre diameter coefficient x is less than or equal to 1 and where the theoretical fibre diameter coefficient x is greater than or equal to 0.25; and
    iii. the sum of the theoretical cover coefficient TCC A and TCC B is greater than or equal to 50%,

    wherein the TCC of each layer is calculated according to the following formula:

$$TCC \% = (4 * m * 100\%) / (\pi * \rho * d)$$

based on (TCC) % = d * L * 100%; L= 4V / $\pi d^2$; V = m / $\rho$
wherein: d is median fibre diameter of a layer (m)

L is length of fibre in 1 $m^2$ of textile ($m/m^2$)
V is volume of fibre in 1 $m^2$ of textile ($m^3/m^2$)
m is mass of fibre in 1 $m^2$ of textile **($g/m^2$)**
$\rho$ is fibre density (mass g/ volume $m^3$)
**characterised in that:**
majority of the fibers of the first barrier layer A and majority of the fibers of the second barrier layer B are meltblown fibers.

2. Nonwoven textile according to claim 1 **characterised in that** the theoretical fibre diameter coefficient x is greater than or equal to 0.3.

3. Nonwoven textile according to any of the preceding claims **characterised in that** the theoretical cover coefficient TCC of each of the individual layers A and/or B is at least 20%, preferably at least 25%, preferably at least 30%.

4. Nonwoven textile according to any of the preceding claims **characterised in that** the theoretical cover coefficient TCC of layer A and/or layer B is less than or equal to 800%, preferably less than or equal to 600%, preferably less than or equal to 400%, preferably less than or equal to 200%.

5. Nonwoven textile according to any of the preceding claims **characterised in that** the layer A and/or both layers A and B contain at least 10% of fibres with a fibre diameter less than 1 micron, preferably up to 20% of fibres with a fibre diameter less than 1 micron, preferably up to 25% of fibres with a fibre diameter less than 1 micron, preferably up to 50% of fibres with a fibre diameter less than 1 micron, preferably up to 100% of fibres with a fibre diameter less than 1 micron.

6. Nonwoven textile according to any of the preceding claims **characterised in that** the layer A and/or both layers A and B contain fibres with a fibre diameter greater than 200 nm, preferably greater than 500 nm.

7. Nonwoven textile according to any of claims 1-5 **characterised in that** it contains one or more layers D, whilst at least one of the D layers is in direct contact with the layer A or with the layer B.

8. Nonwoven textile according to any of claims 1-5 **characterised in that** the main component of fibres in the layer A and/or the layer B contains a compound belonging to a group of polyolefins or polyesters or biopolymers or polyamides or copolymers of the aforementioned groups.

9. Nonwoven textile according to claim 8 **characterised in that** the main component of fibres in the layer A and/or the layer B contains a compound from a group containing polypropylene, polyethylene, PET, PBT, PTT, PLA or copolymers of the aforementioned compounds.

10. Nonwoven textile according to any of claims 1-5 **characterised in that** the main component of fibres in the layer A and/or B contains a blend of compounds.

11. Nonwoven textile according to any of claims 1-5 **characterised in that** it contains several independent pairs of barrier layers A, B.

12. Absorptive hygiene product containing a nonwoven textile according to any of the preceding claims.

13. Product with absorptive properties containing a permeable layer, a sorptive core and a barrier nonwoven textile **characterised in that** it contains the barrier nonwoven textile according to any of the claims 1 to 12.

14. Protective garment **characterised in that** it contains the barrier nonwoven textile according to any of the claims 1 to 11.

**Patentansprüche**

1. Ungewebte Textilie mit Barriereeigenschaften, umfassend

   a. eine erste Barriereschicht A, die einen theoretischen Bedeckungskoeffizienten TCC A aufweist und aus Fasern mit einem innerhalb der Schicht ermittelten Medianwert dAm des Faserdurchmessers besteht; und
   b. eine zweite Barriereschicht B, die einen theoretischen Bedeckungskoeffizienten TCC B aufweist und aus Fasern mit einem innerhalb der Schicht ermittelten Medianwert dBm des Faserdurchmessers besteht;

   wobei:

   i. die erste Barriereschicht A und die zweite Barriereschicht B sich in einer direkten gegenseitigen Berührung befinden;
   ii. die Medianwerte der Faserdurchmesser innerhalb der ersten und zweiten Schicht einen theoretischen Koeffizienten x = (dBm-dAm)/dAm der Faserdurchmesser aufweisen, wobei der theoretische Koeffizient x der Faserdurchmesser kleiner als oder gleich 1 ist und wobei der theoretische Koeffizient x der Faserdurchmesser grösser als oder gleich 0,25 ist; und
   iii. die Summe der theoretischen Bedeckungskoeffizienten TCC A und TCC B grösser als oder gleich 50 % ist,

   wobei der Koeffizient TCC jeder der Schichten nach der folgenden Formel berechnet wird:

   $$\text{TCC \%} = (4 * m * 100\%) / (\pi * \rho * d)$$

   unter Bezugnahme von den grundlegenden Formeln (TCC) % = d * L * 100%; L = 4V / $\pi d^2$; V = m / p
   wobei: d (m) ein Medianwert der Faserdurchmesser innerhalb einer Schicht ist,

   L (m/m$^2$) die Länge einer Faser innerhalb von 1 m$^2$ der Textilie ist,
   V (m$^3$/m$^2$) das Volumen von Fasern innerhalb von 1 m$^2$ der Textilie ist,
   m (g/ m$^2$) das Gewicht von Fasern innerhalb von 1 m$^2$ der Textilie ist,
   p (Gew. g/Vol. m$^3$) die Dichte von Fasern ist,

   **dadurch gekennzeichnet, dass**
   der überwiegende Anteil der in der ersten Barriereschicht A enthaltenen Fasern sowie der überwiegende Anteil der in der zweiten Barriereschicht B enthaltenen Fasern Meltblown-Fasern sind.

2. Ungewebte Textilie nach Anspruch 1, **dadurch gekennzeichnet, dass** der theoretische Koeffizient x der Faserdurchmesser grösser als oder gleich 0,3 ist.

3. Ungewebte Textilie nach einem der vorhergehenden Ansprüche, **dadurch gekennzeichnet, dass** der theoretische Bedeckungskoeffizient TCC jeder der einzelnen Schichten A und/oder B mindestens 20 %, vorzugsweise mindestens 25 %, vorzüglicherweise 30 % beträgt.

4. Ungewebte Textilie nach einem der vorhergehenden Ansprüche, **dadurch gekennzeichnet, dass** der theoretische Bedeckungskoeffizient TCC der Schicht A und/oder der Schicht B kleiner als oder gleich 800 %, vorzugsweise kleiner als oder gleich 600 %, vorzüglicherweise kleiner als oder gleich 400 % und am besten kleiner als oder gleich 200 % ist.

5. Ungewebte Textilie nach einem der vorhergehenden Ansprüche, **dadurch gekennzeichnet, dass** die Schicht A und/oder beide Schichten A und B Fasern mit einem Anteil von mindestens 10 %, deren Durchmesser weniger als 1 Mikrometer beträgt, vorzugsweise Fasern mit einem Anteil von bis 20 %, deren Durchmesser weniger als 1 Mikrometer beträgt, vorzüglicherweise Fasern mit einem Anteil von bis 25 %, deren Durchmesser weniger als 1 Mikrometer beträgt, vorzüglicherweise Fasern mit einem Anteil von bis 50 %, deren Durchmesser weniger als 1 Mikrometer beträgt, am besten Fasern mit einem Anteil von bis 100 %, deren Durchmesser weniger als 1 Mikrometer beträgt, enthält, bzw. enthalten.

6. Ungewebte Textilie nach einem der vorhergehenden Ansprüche, **dadurch gekennzeichnet, dass** die Schicht A und/oder beide Schichten A und B Fasern enthält, bzw. enthalten, deren Durchmesser grösser ist als 200 nm, vorzugsweise grösser ist als 500 nm.

**7.** Ungewebte Textilie nach einem der Ansprüche 1-5, **dadurch gekennzeichnet, dass** sie eine oder mehrere Schichten D enthält, wobei mindestens eine der Schichten D in direkter Berührung mit der Schicht A oder mit der Schicht B steht.

**8.** Ungewebte Textilie nach einem der Ansprüche 1-5, **dadurch gekennzeichnet, dass** der Hauptbestandteil der in der Schicht A und/oder in der Schicht B enthaltenen Fasern eine Verbindung umfasst, die zu einer Gruppe von Polyolefinen oder Polyestern oder Biopolymeren oder Polyamiden oder Kopolymeren der oben angeführten Gruppen gehört.

**9.** Ungewebte Textilie nach Anspruch 8, **dadurch gekennzeichnet, dass** der Hauptbestandteil der in der Schicht A und/oder in der Schicht B enthaltenen Fasern eine Verbindung umfasst, die zu einer Gruppe gehört, die aus Polypropylen, Polyethylen, PET, PBT, PTT, PLA oder Kopolymeren der oben angeführten Verbindungen besteht.

**10.** Ungewebte Textilie nach einem der Ansprüche 1-5, **dadurch gekennzeichnet, dass** der Hauptbestandteil der in der Schicht A und/oder in der Schicht B enthaltenen Fasern ein Gemisch von Verbindungen umfasst.

**11.** Ungewebte Textilie nach einem der Ansprüche 1-5, **dadurch gekennzeichnet, dass** sie mehrere unabhängige Paare von Barriereschichten A und B enthält.

**12.** Ein absorbierendes Hygieneprodukt, enthaltend eine ungewebte Textilie nach einem der vorhergehenden Ansprüche.

**13.** Ein Produkt mit absorbierenden Eigenschaften, enthaltend eine durchlässige Schicht, einen saugfähigen Kern sowie eine ungewebte Textilie mit Barriereeigenschaften, **dadurch gekennzeichnet, dass** es die ungewebte Textilie mit Barriereeigenschaften nach einem de Ansprüche 1 bis 12 enthält.

**14.** Eine Schutzkleidung, **dadurch gekennzeichnet, dass** sie die ungewebte Textilie mit Barriereeigenschaften nach einem der Ansprüche 1 bis 11 enthält.

**Revendications**

**1.** Textile non-tissé à propriétés barrières qui contient :

a. une première couche barrière A à un coefficient de couverture théorique TCC A, et consistant en fibres à un diamètre médian de fibres dAm dans la couche ; et
b. une deuxième couche barrière B à un coefficient de couverture théorique TCC B, et consistant en fibres à un diamètre médian de fibres dBm dans la couche ;
c. une troisième couche D ;

**où**

i. la première couche barrière A et la deuxième couche barrière B sont en contact direct mutuel ;
ii. les diamètres médians de fibres dans la première et la deuxième couche ont un coefficient de diamètre de fibre théorique x = (dBm-dAm)/dAm, où le coefficient de diamètre de fibre théorique x est égal ou inférieur à 1 et où le coefficient de diamètre de fibre théorique est égal ou supérieur à 0.25 ; et
iii. le total du coefficient de couverture théorique TCC A et TCC B est égal ou supérieur à 50%,

où le TCC de chaque couche est calculé selon la formule suivante :

$$TCC\% = (4 * m * 100\%) / (\pi * \rho * d)$$

sur la base de (TCC) % = d $^*$ L $^*$ 100% ; L = 4V / $\pi d^2$; V = m / $\rho$
où : d est le diamètre médian de fibre d'une couche (m)

L est la longueur de fibre en 1 m$^2$ de textile (m/m$^2$)
V est le volume de fibre en 1 m$^2$ de textile (m$^3$/m$^2$)
m est la masse de fibres en 1 m$^2$ de textile (g/m$^2$)
$\rho$ est la densité de fibre (masse g/ volume m$^3$)

**caractérisé en ce que** la plupart des fibres de la première couche barrière A et la plupart des fibres de la deuxième couche barrière B sont des fibres obtenus par fusion-soufflage.

**2.** Textile non-tissé selon la revendication 1, **caractérisé en ce que** le coefficient de diamètre de fibre théorique x est égal ou supérieur à 0.3.

**3.** Textile non-tissé selon l'une quelconque des revendications précédentes, **caractérisé en ce que** le coefficient de couverture théorique TCC de chacune des couches individuelles A et/ou B est au moins 20%, préférablement au moins 25%, préférablement au moins 30%.

**4.** Textile non-tissé selon l'une quelconque des revendications précédentes, **caractérisé en ce que** le coefficient de couverture théorique TCC de la couche A et/ou de la couche B est égal ou inférieur à 800%, préférablement égal ou inférieur à 600%, préférablement égal ou inférieur à 400%, préférablement égal ou inférieur à 200%.

**5.** Textile non-tissé selon l'une quelconque des revendications précédentes, **caractérisé en ce que** la couche A et/ou les deux couches A et B contiennent au moins 10% de fibres à un diamètre de fibre inférieur à 1 micron, préférablement jusqu'à 20% de fibres à un diamètre de fibre inférieur à 1 micron, préférablement jusqu'à 25% de fibres à un diamètre de fibre inférieur à 1 micron, préférablement jusqu'à 50% de fibres à un diamètre de fibre inférieur à 1 micron, préférablement jusqu'à 100% de fibres à un diamètre de fibre inférieur à 1 micron.

**6.** Textile non-tissé selon l'une quelconque des revendications précédentes, **caractérisé en ce que** la couche A et/ou les deux couches A et B contiennent des fibres à diamètre supérieur à 200 nm, préférablement supérieur à 500 nm.

**7.** Textile non-tissé selon l'une quelconque des revendications 1-5, **caractérisé en ce qu'**il contient une ou plusieurs couches D, alors qu'au moins une des couches D est en contact direct avec la couche A ou la couche B.

**8.** Textile non-tissé selon l'une quelconque des revendications 1-5, **caractérisé en ce que** le composant principal des fibres dans la couche A et/ou dans la couche B contient un composé qui appartient au groupe de polyoléfines ou polyesters ou biopolymères ou polyamides ou copolymères des groupes mentionnés ci-dessus.

**9.** Textile non-tissé selon la revendication 8, **caractérisé en ce que** le composant principal dans la couche A et/ou la couche B contient un composé d'un groupe contenant polypropylène, polyéthylène, PET, PBT, PTT, PLA ou des copolymères des composés mentionnés ci-dessus.

**10.** Textile non-tissé selon l'une quelconque des revendications 1-5, **caractérisé en ce que** le composant principal des fibres dans la couche A et/ou B contient un mélange de composés.

**11.** Textile non-tissé selon l'une quelconque des revendications 1-5, **caractérisé en ce qu'**il contient plusieurs paires indépendantes des couches barrières A, B.

**12.** Produit d'hygiène absorbant, contenant un textile non-tissé selon l'une quelconque des revendications précédentes.

**13.** Produit ayant des propriétés d'absorption contenant une couche perméable, un noyau sorbant et un textile non-tissé barrière **caractérisé en ce qu'**il contient le textile non-tissé barrière selon l'une quelconque des revendications 1 à 12.

**14.** Vêtement de protection **caractérisé en ce qu'**il contient un textile non-tissé barrière selon l'une quelconque des revendications 1 à 11.

Fig. 1

Fig. 2

Fig. 3a

Fig. 3b

Fig. 4.

a) Example 1

b) Example 2

c) Example 3 + 4

d) Example 5

e) Example 6

f) Example 7

g) Example 8

h) Example 9-13

Fig.5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2019875 A
- US 7585437 B
- EP 1639173 A
- US 2009232920 A
- WO 2011100407 A, Procter & Gamble
- US 2005215155 A
- US 6632385 B
- US 6803103 B
- US 20060057921 A
- WO 2014044235 A